# EUROPEAN PATENT APPLICATION

(11) **EP 4 299 743 A1**
(43) Date of publication of application: **03.01.2024**
(21) Application number: 22759568.3
(22) Date of filing: 21.02.2022
(51) Int. Cl.: C12N 15/113, A23K 20/153, A23L 33/13, A61K 31/7088, A61K 31/712, A61K 48/00, A61P 3/10, A61P 9/00, A61P 9/04, A61P 9/10, A61P 13/12, A61P 19/02, A61P 19/08, A61P 21/00, A61P 29/00, A61P 43/00, C12N 5/10

(54) **NUCLEIC ACID MEDICINE EXPRESSING SPLICING VARIANT OF MYOSTATIN**

(30) Priority: 26.02.2021 JP 2021029890
(71) Applicant: KNC Laboratories Co., Ltd., Kobe-shi, Hyogo 650-0047 (JP)
(72) Inventor: MATSUO, Masafumi, Kobe-shi, Hyogo 651-2180 (JP); MAETA, Kazuhiro, Kobe-shi, Hyogo 651-2180 (JP)
(74) Representative: Hoffmann Eitle
(86) International application number: PCT/JP2022/006907
(87) International publication number: WO 2022/181532

(57) **Abstract**

A method of inhibiting the function of myostatin is provided.

An antisense oligonucleotide of 15-30 bases or a salt or a solvate thereof, wherein the antisense oligonucleotide has a nucleotide sequence complementary to a target sequence in exon 3 of the myostatin gene and is capable of allowing the expression of a splicing variant of myostatin. A pharmaceutical drug, a food, a feed, an agent for promoting myocyte proliferation and/or hypertrophy, an agent for increasing muscle mass and/or suppressing muscle weakness, an agent for switching the splicing of the myostatin gene from production of myostatin to production of a splicing variant thereof, an agent for decreasing myostatin signaling, and an anticancer agent, each of which comprises the above antisense oligonucleotide or a salt or a solvate thereof.

## Description

### TECHNICAL FIELD

The present invention relates to a nucleic acid drug which allows expression of a splicing variant of myostatin.

### BACKGROUND ART

Myostatin is one of TGF-β cell proliferation factors and is a protein encoded by the myostatin gene. Myostatin has an effect of inhibiting myocyte growth/hypertrophy. Therefore, since inhibiting the function of myostatin promotes myocyte growth/hypertrophy, myostatin has been attracting attention as a target of muscular atrophy therapy (Non-Patent Document No. 1). Moreover, decrease in myostatin expression inhibits cancer cell proliferation (Non-Patent Document No. 2). These results suggest that decrease in myostatin level and inhibition of myostatin signaling are effective in cancer treatment. Further, since expression levels of myostatin are elevated in type 2 diabetes patients, it is thought that there is some relation between myostatin and diabetes (Non-Patent Document No. 3). From what have been described above, it is believed that inhibition of myostatin signaling would be effective for suppression of diabetes or inhibition of its progression.

To date, antibodies to myostatin or antisense oligonucleotides (ASOs), etc. which allow the production of out-of-frame mRNA by regulating the splicing of the myostatin gene have been developed (Non-Patent Documents No. 4 and No. 5). However, no clinically useful method of inhibiting myostatin has yet been established.

### PRIOR ART LITERATURE

### Non-Patent Documents

Non-Patent Document No. 1: Bogdanovich et al., Nature, 2002, 420;418-421
Non-Patent Document No. 2: Han et al., Redox Biol. 2018, 19;412-4128
Non-Patent Document No. 3: Palsgaard et al. 2009, 4;e6575
Non-Patent Document No. 4: St Andre et al. Skeletal Muscle 2017,7;25
Non-Patent Document No. 5: Kang et al. Mol Ther 2011,19;159-64

### DISCLOSURE OF THE INVENTION

### PROBLEM FOR SOLUTION BY THE INVENTION

It is an object of the present invention to provide a method of inhibiting the function of myostatin.

### MEANS TO SOLVE THE PROBLEM

The myostatin (MSTN) gene has a simple structure with three exons. Although it was thought that only one type of mRNA is produced from MSTN gene, the present inventors have revealed that a splicing variant mRNA is newly produced upon activation of a cryptic splice acceptor site in 3' untranslated region of MSTN gene (WO2020/179571). Further, the present inventors have also revealed that an isoform encoded by this variant mRNA has an effect of inhibiting myostatin. This time, the present inventors have searched for a nucleic acid drug which alters the mode of splicing of the human myostatin gene to thereby promote the production of a novel mRNA. The present inventors have hence synthesized and screened various types of ASOs complementary to sequences of exon 3, using modified amino acids ENA (2'-O,4'-C-Ethylene-bridged Nucleic Acids) as monomers, and identified an ASO which promotes the production of a splicing variant of interest most efficiently. Further, the present inventors have confirmed that a myostatin isoform of interest is produced in cells by introduction of these ASOs. Inhibition of the function of myostatin is a target of muscular atrophy therapy, and a number of myostatin inhibitors have been developed. However, a myostatin inhibitor like the ASO of the present invention which allows the production of a splicing variant of myostatin is the first type ever developed in the world.

A summary of the present invention is described as below.
(1) An antisense oligonucleotide of 15-30 bases or a salt or a solvate thereof, wherein the antisense oligonucleotide has a nucleotide sequence complementary to a target sequence in exon 3 of the myostatin gene and is capable of allowing the expression of a splicing variant of myostatin.
(2) The antisense oligonucleotide or a salt or a solvate thereof of (1) above, wherein the nucleotide sequence of exon 3 of the myostatin gene is the nucleotide sequence as shown in SEQ ID NO: 1; the target sequence in exon 3 of the myostatin gene is the sequence of the region of nucleotide Nos. 10-33 of the nucleotide sequence as shown in SEQ ID NO: 1; and the nucleotide sequence of said target sequence is represented by SEQ ID NO: 2.
(3) The antisense oligonucleotide or a salt or a solvate thereof of (1) or (2) above, wherein the nucleotide sequence of the antisense oligonucleotide comprises a sequence consisting of at least 15 consecutive nucleotides in any one of the sequences as shown in SEQ ID NO: 24, 30, 31, 27, 32, 33 or 28 (wherein "t" may be "u", and "u" may be "t").
(4) The antisense oligonucleotide or a salt or a solvate thereof of any one of (1) to (3) above, wherein the antisense oligonucleotide has 18 bases.
(5) The antisense oligonucleotide or a salt or a solvate thereof of (4) above, wherein the nucleotide sequence of the antisense oligonucleotide is any one of the sequences as shown in SEQ ID NO: 24, 30, 31, 27, 32, 33 or 28 (wherein "t" may be "u", and "u" may be "t").
(6) The antisense oligonucleotide or a salt or a solvate thereof of any one of (1) to (5) above, wherein at least one nucleotide is modified.
(7) The antisense oligonucleotide or a salt or a solvate thereof of (6) above, wherein the sugar constituting the modified nucleotide is D-ribofuranose and the hydroxy group at 2'-position of D-ribofuranose is modified.
(8) The antisense oligonucleotide or a salt or a solvate thereof of (7) above, wherein D-ribofuranose is 2'-O-alkylated and/or 2'-O,4'-C-alkylenated.
(9) A pharmaceutical drug comprising the antisense oligonucleotide of any one of (1) to (8) above or a pharmaceutically acceptable salt or solvate thereof.
(10) The pharmaceutical drug of (9) above for preventing and/or treating a pathological condition and/or a disease in which myostatin is involved.
(11) The pharmaceutical drug of (10) above, wherein the condition and/or disease in which myostatin is involved is muscular atrophy.
(12) The pharmaceutical drug of (1 1) above, wherein muscular atrophy is at least one selected from the group consisting of muscular dystrophy, myopathy, spinal muscular atrophy, sarcopenia and disuse muscle atrophy.
(13) The pharmaceutical drug of (10) above, wherein the condition and/or disease in which myostatin is involved is a pathological condition and/or a disease in which a therapeutic effect is gained through muscle mass recovery.
(14) The pharmaceutical drug of (13) above, wherein the condition and/or disease in which a therapeutic effect is gained through muscle mass recovery is at least one selected from the group consisting of cancer cachexia, diabetes, cardiovascular diseases, renal diseases and bone diseases.
(15) The pharmaceutical drug of (14) above, wherein the cardiovascular disease is cardiac failure and/or arteriosclerosis; the renal disease is chronic renal failure; and the bone disease is inflammatory arthritis.
(16) A food comprising the antisense oligonucleotide of any one of (1) to (8) above or a salt or a solvate thereof that are acceptable as a food ingredient.
(17) A feed comprising the antisense oligonucleotide of any one of (1) to (8) above or a salt or solvate thereof that are acceptable as a feed ingredient.
(18) An agent for promoting myocyte proliferation and/or hypertrophy, comprising the antisense oligonucleotide or a salt or a solvate thereof of any one of (1) to (8) above.
(19) An agent for increasing muscle mass and/or suppressing muscle weakness, comprising the antisense oligonucleotide or a salt or a solvate thereof of any one of (1) to (8) above.
(20) An agent for switching the splicing of the myostatin gene from production of myostatin to production of a splicing variant thereof, comprising the antisense oligonucleotide or a salt or a solvate thereof of any one of (1) to (8) above.
(21) An agent for decreasing myostatin signaling, comprising the antisense oligonucleotide or a salt or a solvate thereof of any one of (1) to (8) above.
(22) An anticancer agent comprising the antisense oligonucleotide or a salt or a solvate thereof of any one of (1) to (8) above.
(23) A method of preventing and/or treating a pathological condition and/or a disease in which myostatin is involved, comprising administering to a subject an effective amount of the antisense oligonucleotide or a salt or a solvate thereof of any one of (1) to (8) above.
(24) A method of promoting myocyte proliferation and/or hypertrophy, comprising administering to a subject an effective amount of the antisense oligonucleotide or a salt or a solvate thereof of any one of (1) to (8) above.
(25) A method of increasing muscle mass and/or suppressing muscle weakness, comprising administering to a subject an effective amount of the antisense oligonucleotide or a salt or a solvate thereof of any one of (1) to (8) above.
(26) A method of switching the splicing of the myostatin gene from production of myostatin to production of a splicing variant thereof, comprising administering to a subject an effective amount of the antisense oligonucleotide or a salt or a solvate thereof of any one of (1) to (8) above.
(27) A method of decreasing myostatin signaling, comprising administering to a subject an effective amount of the antisense oligonucleotide or a salt or a solvate thereof of any one of (1) to (8) above.
(28) A method of preventing and/or treating cancer, comprising administering to a subject an effective amount of the antisense oligonucleotide or a salt or a solvate thereof of any one of (1) to (8) above.
(29) The antisense oligonucleotide or a salt or a solvate thereof of any one of (1) to (8) above, for use in a method of preventing and/or treating a pathological condition and/or a disease in which myostatin is involved.
(30) The antisense oligonucleotide or a salt or a solvate thereof of any one of (1) to (8) above, for use in a method of promoting myocyte proliferation and/or hypertrophy.
(31) The antisense oligonucleotide or a salt or a solvate thereof of any one of (1) to (8) above, for use in a method of increasing muscle mass and/or suppressing muscle weakness.
(32) The antisense oligonucleotide or a salt or a solvate thereof of any one of (1) to (8) above, for use in a method of switching the splicing of the myostatin gene from production of myostatin to production of a splicing variant thereof.
(33) The antisense oligonucleotide or a salt or a solvate thereof of any one of (1) to (8) above, for use in a method of decreasing myostatin signaling.
(34) The antisense oligonucleotide or a salt or a solvate thereof of any one of (1) to (8) above, for use in a method of preventing and/or treating cancer.
(35) Use of the antisense oligonucleotide or a salt or a solvate thereof of any one of (1) to (8) above, for preventing and/or treating a pathological condition and/or a disease in which myostatin is involved.
(36) Use of the antisense oligonucleotide or a salt or a solvate thereof of any one of (1) to (8) above, for promoting myocyte proliferation and/or hypertrophy.
(37) Use of the antisense oligonucleotide or a salt or a solvate thereof of any one of (1) to (8) above, for increasing muscle mass and/or suppressing muscle weakness.
(38) Use of the antisense oligonucleotide or a salt or a solvate thereof of any one of (1) to (8) above, for switching the splicing of the myostatin gene from production of myostatin to production of a splicing variant thereof.
(39) Use of the antisense oligonucleotide or a salt or a solvate thereof of any one of (1) to (8) above, for decreasing myostatin signaling.
(40) Use of the antisense oligonucleotide or a salt or a solvate thereof of any one of (1) to (8) above, for preventing and/or treating cancer.

The ASO of the present invention modifies the splicing of the MSTN gene to promote the production of a splicing variant of myostatin, thereby inducing the expression of a novel isoform of myostatin. The present inventors have already filed a patent application for this novel isoform as a myostatin inhibitor protein (WO2020/179571). Therefore, the ASO of the present invention may be a novel agent for myostatin inhibition. By administering the ASO of the present invention to human, the function of myostatin is inhibited to enable the prevention/treatment of muscular atrophy. Specifically, the ASO of the present invention is believed to be applicable to treatment of such diseases as muscular dystrophy and myopathy whose cardinal symptom is muscular atrophy. Further, the ASO of the present invention is applicable to prevention and/or treatment of a wide variety of pathological conditions and/or diseases in which a therapeutic effect can be expected from muscle mass recovery (e.g., sarcopenia, cancer cachexia, diabetes and the like).

### EFFECT OF THE INVENTION

It is possible to inhibit myostatin signaling by promoting the production of a splicing variant of myostatin to induce the expression of an isoform of myostatin.

The present specification encompasses the contents disclosed in the specification and/or the drawings of Japanese Patent Application No. 2021-029890 based on which the present patent application claims priority.

### BRIEF DESCRIPTION OF THE DRAWINGS

[Fig. 1] Search for ASOs Which Promote Alternative Splicing
   A schematic diagram of ASO is shown which switches the splicing of MSTN pre-mRNA from production of MSTN to production of MSTN-b. Numbers represent the exons of MSTN pre-mRNA, respectively. MSTN has the full-length of exon 3, whereas MSTN-b has exon 3s which is part of exon 3.
[Fig. 2] ASOs Complementary to Sequences in Intron 2 or Exon 3
   Fifty-four ASOs were prepared against MSTN pre-mRNA. Mark "■" indicates recognition sites of ASOs complementary to MSTN pre-mRNA. Numbers represent the exons of MSTN pre-mRNA, respectively.
[Fig. 3] Comparison of Efficacies of ASOs (MSTN_Ex3_BP, MSTN Ex3_SS, MSTN_Ex3_Fox1, MSTN_Ex3_LESE12, MSTN_Ex3_LESE3 and MSTN_Ex3_LESE4)
   (A) Recognition sites of 6 ASOs against intron 2 or exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.

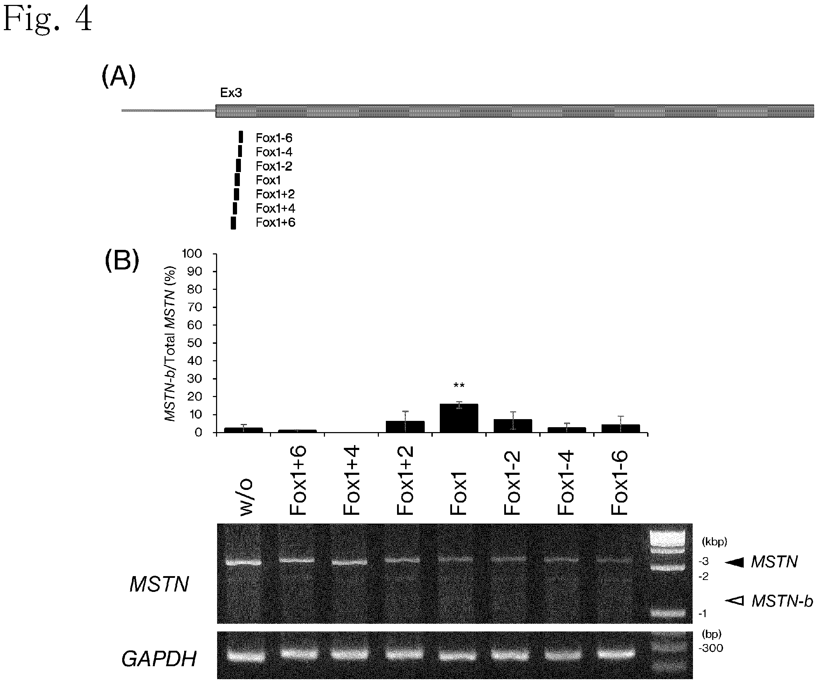

[Fig. 4] Comparison of Efficacies of ASOs (MSTN_Ex3_Fox1+6, MSTN_Ex3_Fox1+4, MSTN_Ex3_Fox1+2, MSTN_Ex3Fox1, MSTN_Ex3_Fox1-2, MSTN_Ex3_Fox1-4 and MSTN_Ex3_Fox1-6)
   (A) Recognition sites of 7 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized. **P<0.01.

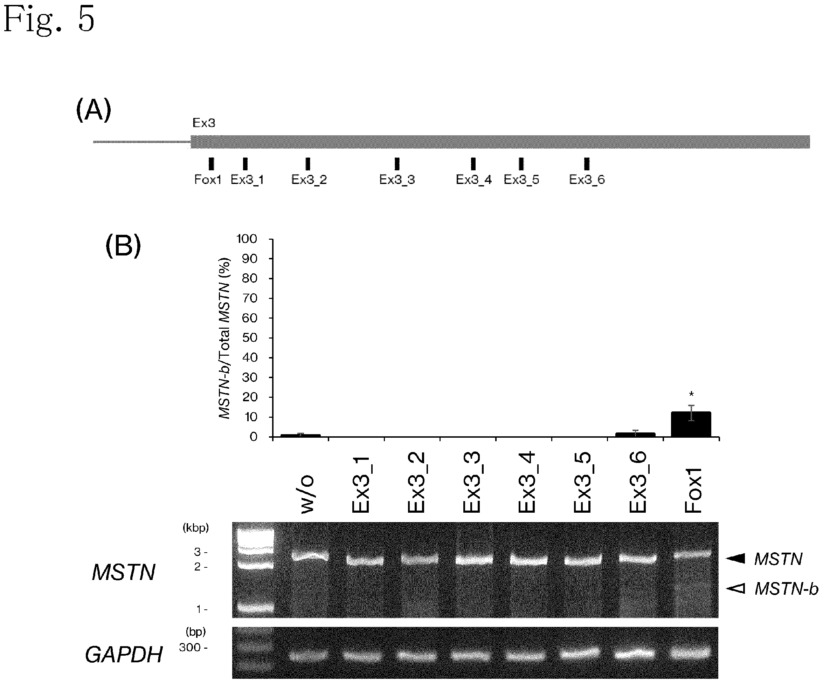

[Fig. 5] Comparison of Efficacies of ASOs (MSTN_Ex3_Fox1, MSTN Ex3_1, MSTN_Ex3_2, MSTN_Ex3_3, MSTN_Ex3_4, MSTN_Ex3_5 and MSTN_Ex3_6)
   (A) Recognition sites of 7 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized. *P<0.05.

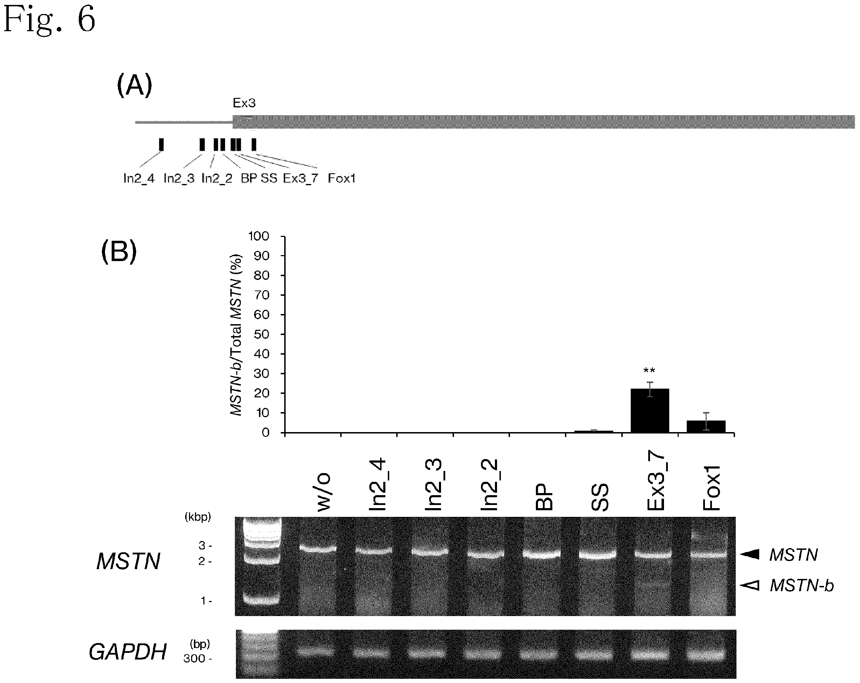

[Fig. 6] Comparison of Efficacies of ASOs (MSTN_In2_4, MSTN_In2_3, MSTN_In2_2, MSTN_Ex3_BP, MSTN_Ex3_SS, MSTN_Ex3_7 and MSTN_Ex3_Fox1)
   (A) Recognition sites of 7 ASOs against intron 2 or exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized. **P<0.01.

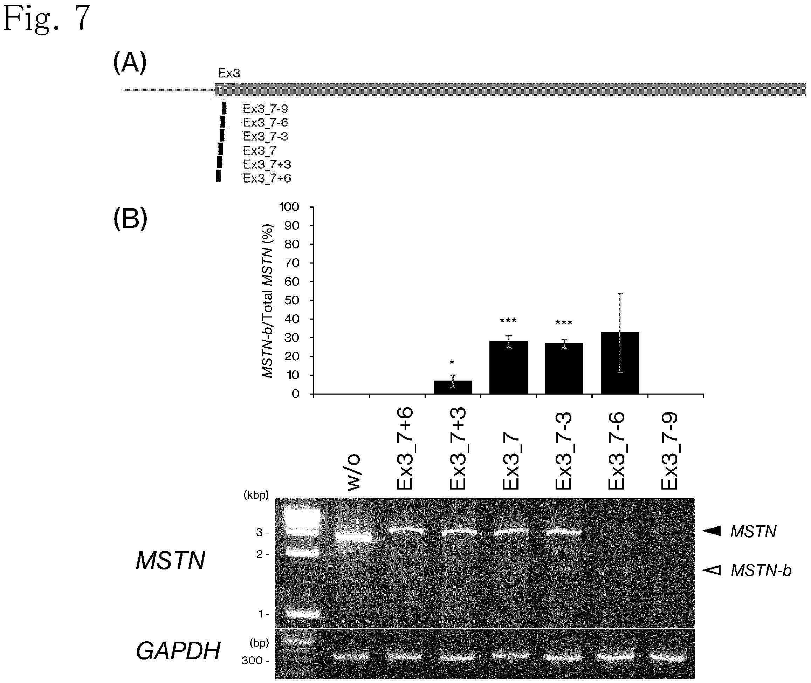

[Fig. 7] Comparison of Efficacies of ASOs (MSTN_Ex3_7+6, MSTN_Ex3_7+3, MSTN_Ex3_7, MSTN_Ex3_7-3, MSTN_Ex3_7-6 and MSTN_Ex3_7-9)
   (A) Recognition sites of 6 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized. *P<0.05, ***P<0.001.

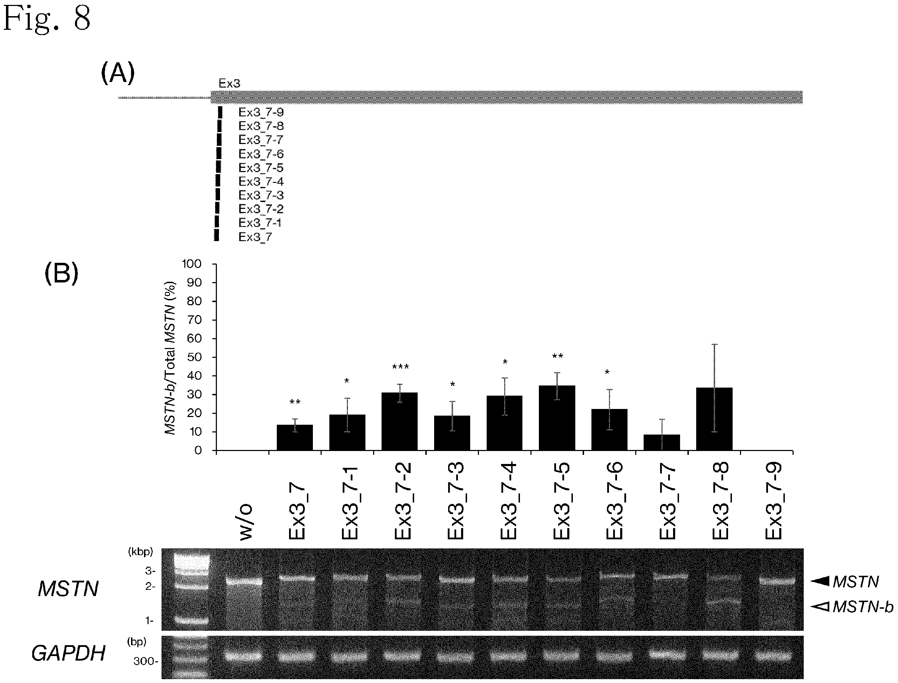

[Fig. 8] Comparison of Efficiencies of ASOs (MSTN_Ex3_7, MSTN_Ex3_7-1, MSTN_Ex3_7-2, MSTN_Ex3_7-3, MSTN_Ex3_7-4, MSTN_Ex3_7-5, MSTN_Ex3_7-6, MSTN_Ex3_7-7, MSTN_Ex3_7-8 and MSTN_Ex3_7-9)
   (A) Recognition sites of 10 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized. *P<0.05, **P<0.01, ***P<0.001.

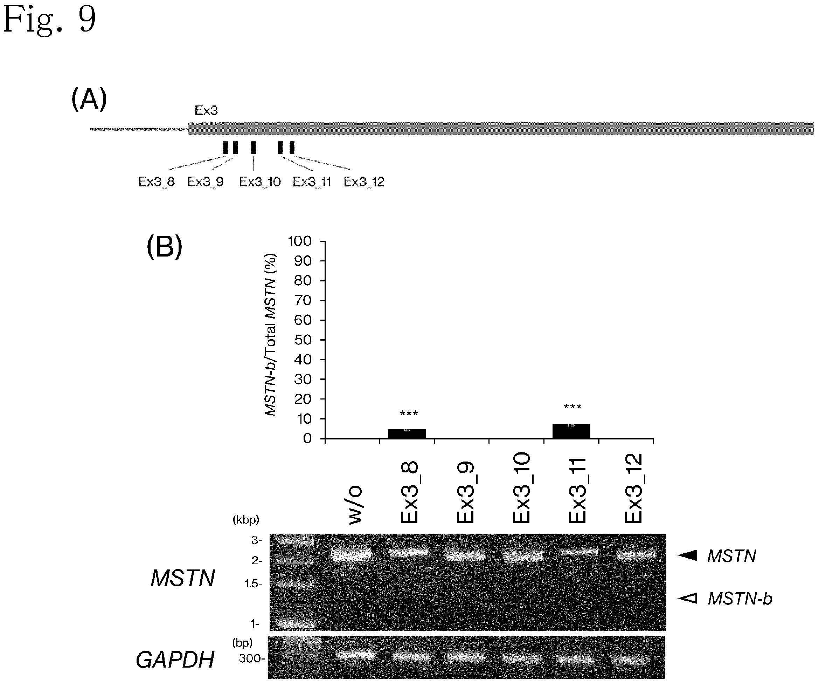

[Fig. 9] Comparison of Efficacies of ASOs (MSTNEx3_8, MSTN Ex3_9, MSTN_Ex3_10, MSTN_Ex3_11 and MSTN_Ex3_12)
   (A) Recognition sites of 5 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.
   ***P<0.001.

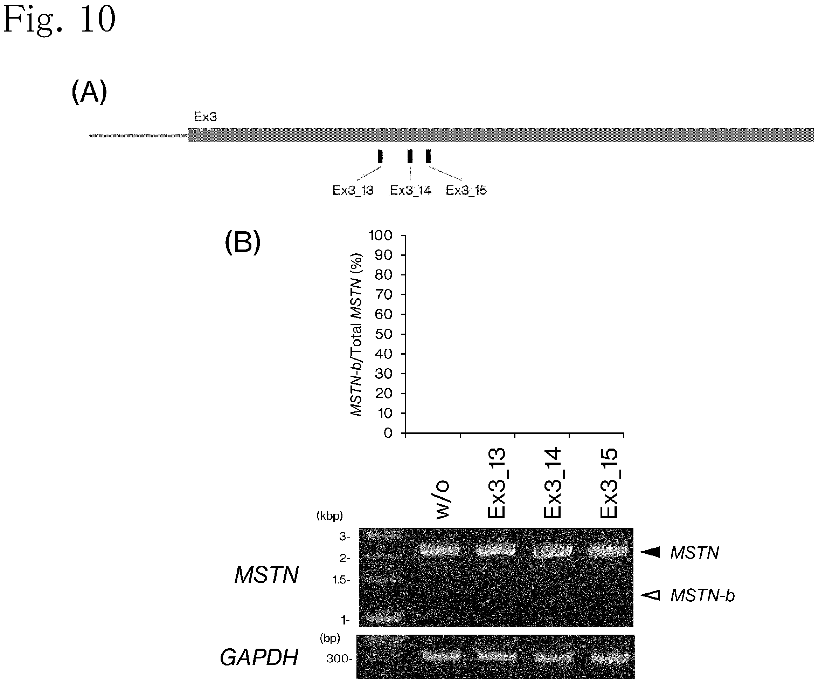

[Fig. 10] Comparison of Efficacies of ASOs (MSTN_Ex3_13, MSTN_Ex3_14 and MSTN_Ex3_15)
   (A) Recognition sites of 3 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.

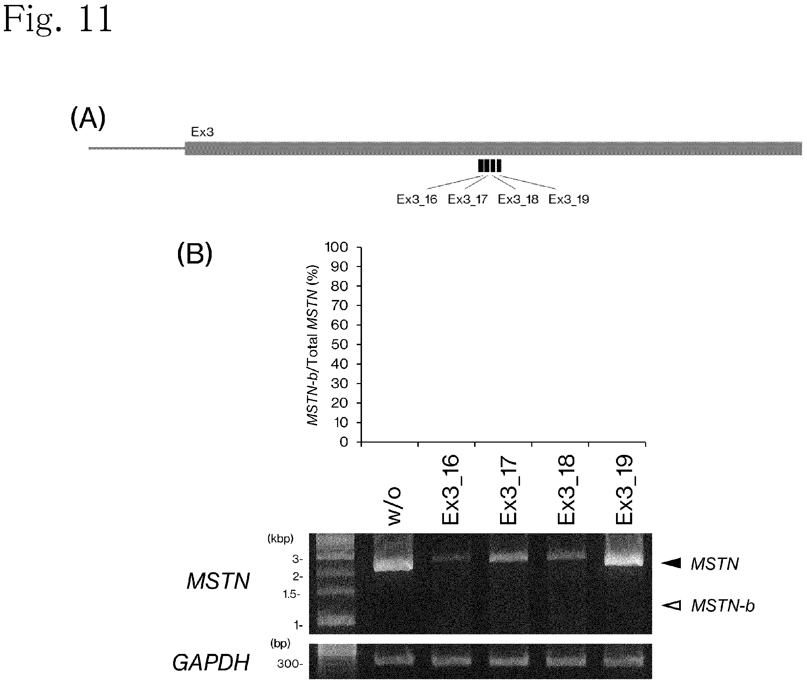

[Fig. 11] Comparison of Efficiencies of ASOs (MSTN_Ex3_16, MSTN_Ex3_17, MSTN_Ex3_18 and MSTN_Ex3_19)
   (A) Recognition sites of 4 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.

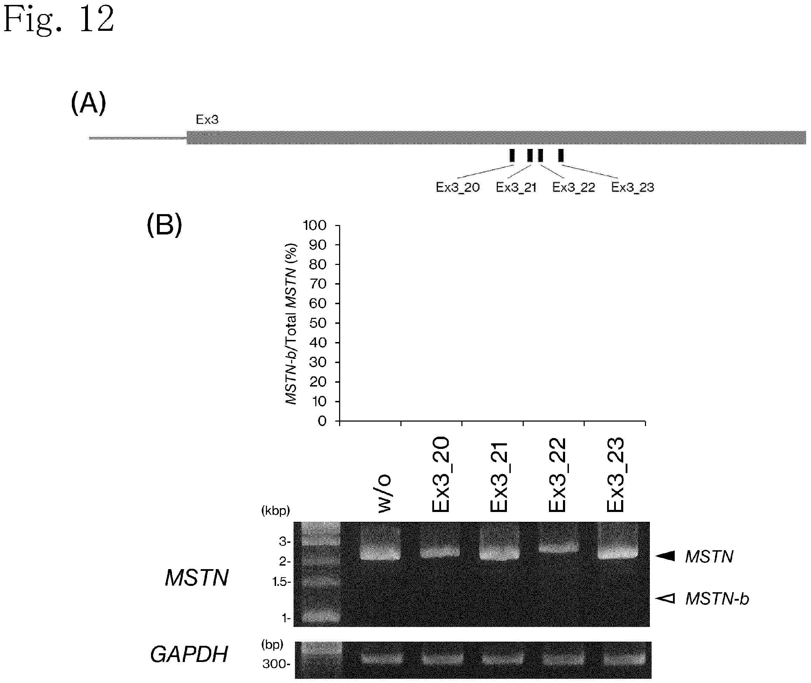

[Fig. 12] Comparison of Efficacies of ASOs (MSTN_Ex3_20, MSTN_Ex3_21, MSTN_Ex3_22 and MSTN_Ex3_23)
   (A) Recognition sites of 4 ASOs against exon 3 of MSTN pre-mRNA are shown. (B) Each ASO was introduced into human rhabdomyosarcoma cells (CRL-2061), and the resultant MSTN mRNA, MSTN-b mRNA, GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.

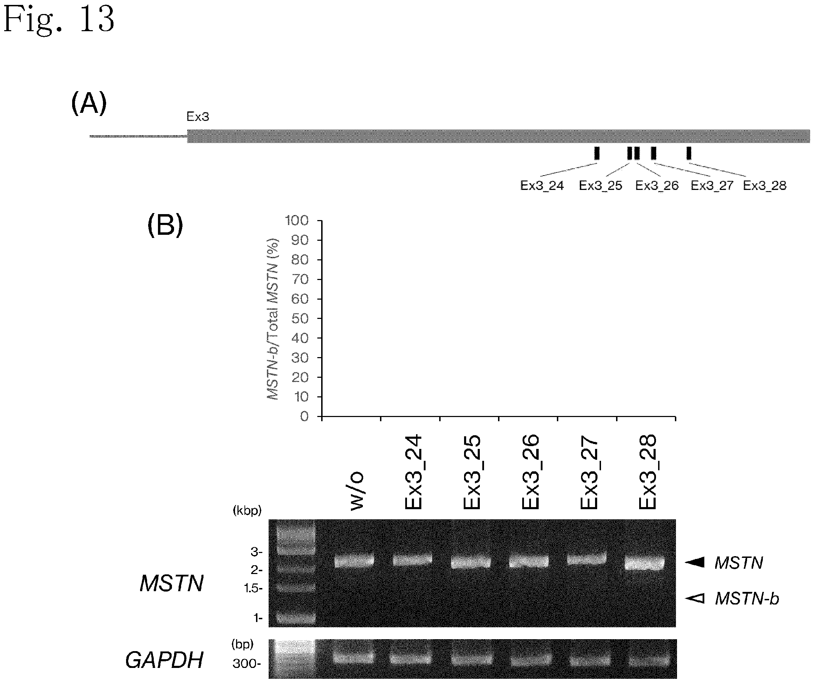

[Fig. 13] Comparison of Efficacies of ASOs (MSTN_Ex3_24, MSTN_Ex3_25, MSTN_Ex3_26, MSTN_Ex3_27 and MSTN_Ex3_28)
   (A) Recognition sites of 5 ASOs against exon 3 of MSTN pre-mRNA are shown. (B) Each ASO was introduced into human rhabdomyosarcoma cells (CRL-2061), and the resultant MSTN mRNA, MSTN-b mRNA and GAPDH mRNA were analyzed by RT-PCR. The results are shown. "w/o" means without ASO treatment. Filled arrowhead represents MSTN and open arrowhead MSTN-b. Graph shows the ratio of MSTN-b to total MSTN. Three independent experiments were conducted and the results were summarized.

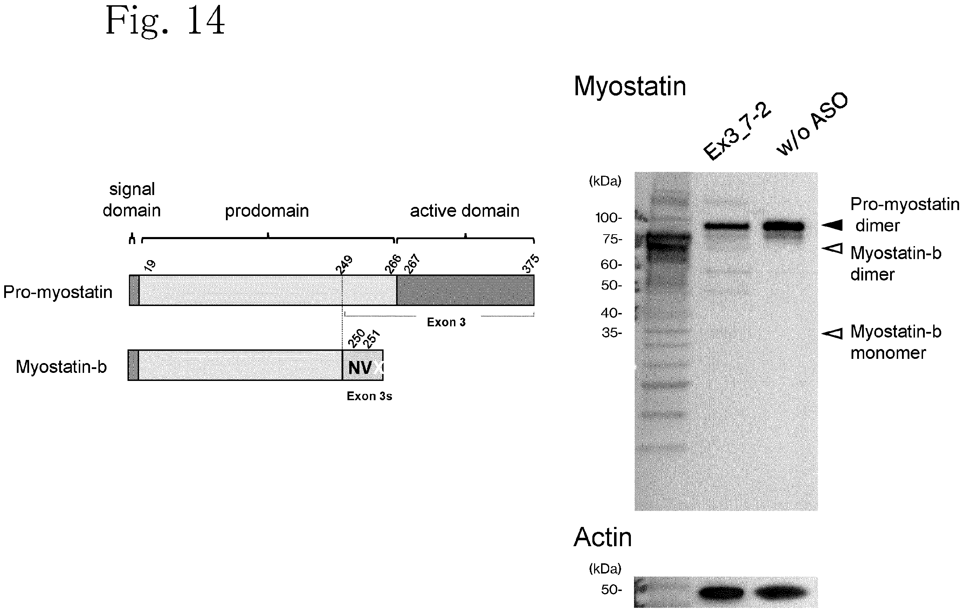

[Fig. 14] Expression of Myostatin-b by ASO Administration
   Schematic diagrams of pro-myostatin and myostatin-b protein are shown (left panel). Each domain and amino acid numbers are shown. Pro-myostatin consists of 375 amino acids in total, and myostatin-b 251 amino acids in total. The sequence of amino acid Nos. 1-250 is common to both pro-myostatin and myostatin-b. The 251^{st} amino acid in myostatin-b is valine. Amino acid Nos. 250-375 are encoded in exon 3 of MSTN, whereas the 250^{th} and 251^{st} amino acids are encoded in exon 3s of MSTN-b. Since mature myostatin is produced as a result of cutting out by protease of the active domain of pro-myostatin translated from MSTN mRNA, no mature myostatin is produced from MSTN-b mRNA that does not retain the region encoding the active domain. Expressions of myostatin and actin after treatment with MSTN_Ex3_7-2 ASO were validated (right panel). Upon ASO treatment, pro-myostatin dimer was decreased, and expression of myostatin-b dimer and myostatin-b monomer was confirmed.

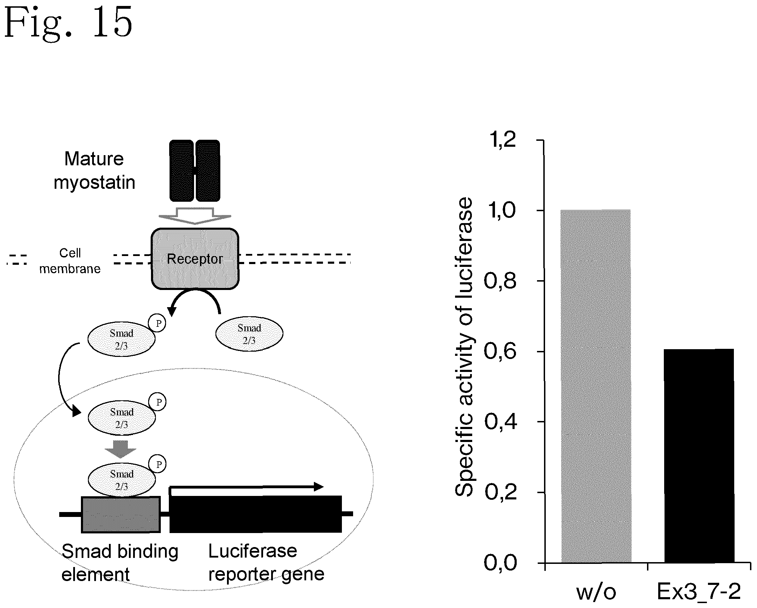

[Fig. 15] Decrease of Myostatin Signaling by ASO Administration
   This is a schematic diagram showing the effect of myostatin on a measuring system for *in vitro* myostatin transcriptional activity (left panel). When myostatin (mature myostatin) is bound to its receptor, transcription factor Smad2/3 is activated, resulting in activation of the transcription of the target gene. In the measuring system for *in vitro* myostatin transcriptional activity, a luciferase reporter gene is used as the target gene of Smad2/3 that is activated by myostatin. Therefore, it is possible to evaluate myostatin signaling by luciferase activity. Briefly, MSTN_Ex3_7-2 ASO was administered to rhabdomyosarcoma cells (CRL-2061), and myostatin signaling was evaluated with the measuring system for *in vitro* myostatin transcriptional activity (right panel). Luciferase activity (luciferase activity/β-galactosidase activity) is shown in relative values with the result of measurement with an extract from cells without ASO treatment (w/o) being taken as 1. Two independent experiments were conducted and the results were summarized.

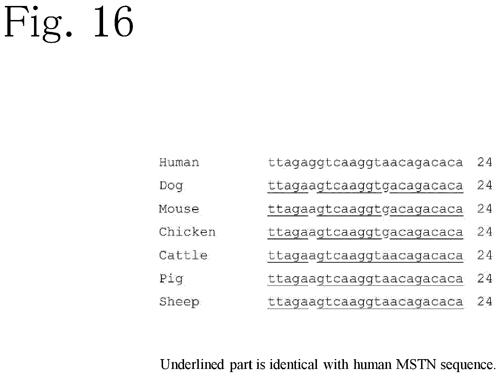

[Fig. 16] Target Sequences of ASOs Highly Preserved Across Species
   An alignment of the nucleotide sequences of human, dog, mouse, chicken, cattle, pig and sheep MSTN exon 3 is shown. Underlined parts are identical with the human sequence.

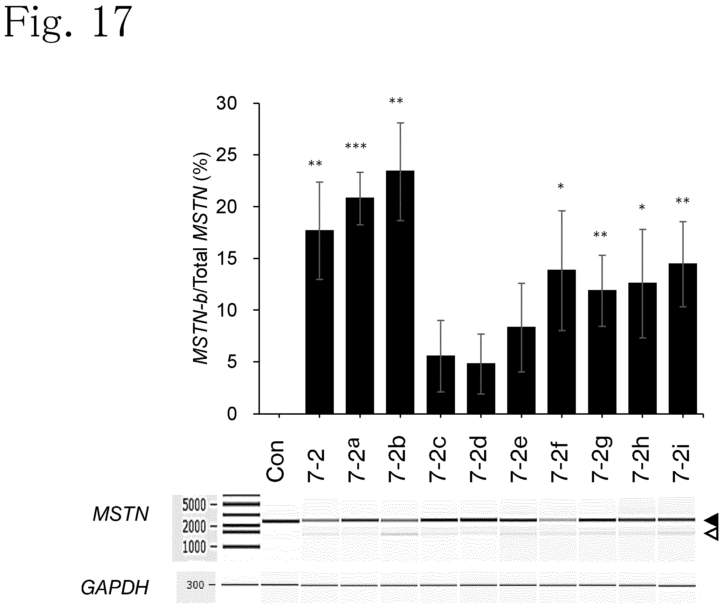

[Fig. 17] Comparison of Efficiencies of ASOs (MSTN_Ex3_7-2a, MSTN_Ex3_7-2b, MSTN_Ex3_7-2c, MSTN_Ex3_7-2d, MSTN_Ex3_7-2e, MSTN_Ex3_7-2f, MSTN_Ex3_7-2g, MSTN_Ex3_7-2h, and MSTN_Ex3_7-2i) in Which the Locations of ENAs in MSTN_Ex3_7-2 Have Been Changed
   Each ASO was introduced into human rhabdomyosarcoma cells (CRL-2061), and the resultant MSTN mRNA, MSTN-b mRNA and GAPDH mRNA were analyzed by RT-PCR. The results are shown. "Con" means without ASO treatment. Filled arrowhead represents MSTN and open arrowhead MSTN-b. Graph shows the ratio of MSTN-b to total MSTN. Three independent experiments were conducted and the results were summarized. *P<0.05, **P<0.01, ***P<0.001.

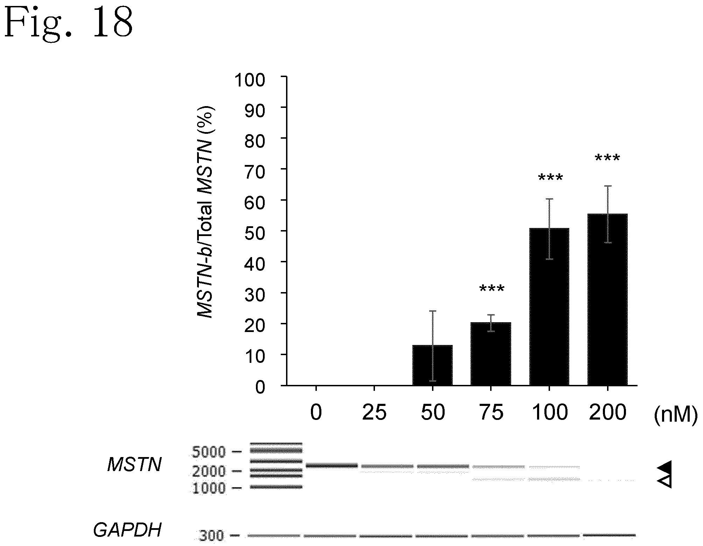

[Fig. 18] Examination of Concentration Dependency of MSTN_Ex3_7-2b.
   ASO was administered to Human rhabdomyosarcoma cells (CRL-2061) at the final concentration of 0, 25, 50, 75, 100 or 200 nM, and the resultant MSTN mRNA, MSTN-b mRNA and GAPDH mRNA were analyzed by RT-PCR. The results are shown. Filled arrowhead represents MSTN and open arrowhead MSTN-b. Graph shows the ratio of MSTN-b to total MSTN. Three independent experiments were conducted and the results were summarized. ***P<0.001.

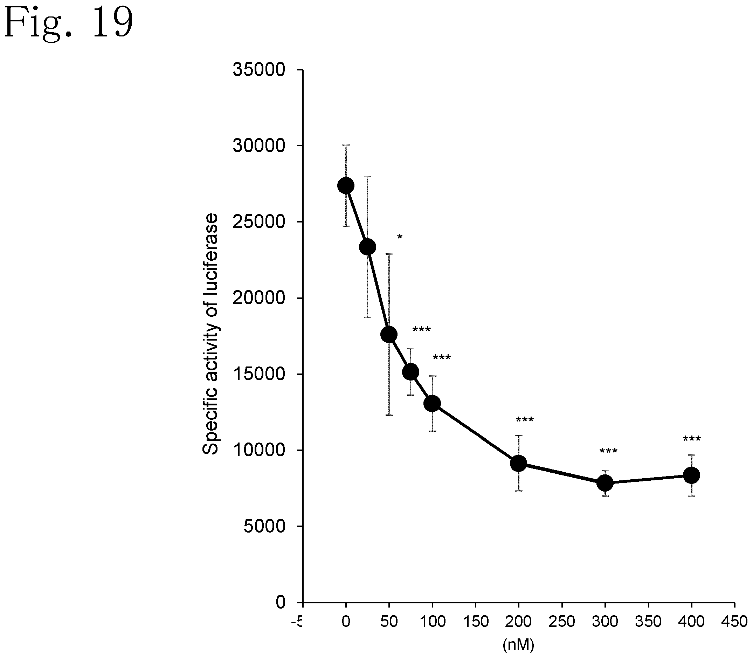

[Fig. 19] Concentration Dependent Decrease of Myostatin Signaling by MSTN_Ex3_7-2b Administration
   ASO was administered to human rhabdomyosarcoma cells (CRL-2061) at the final concentration of 0, 25, 50, 75, 100, 200, 300 or 400 nM. Then, myostatin signaling was evaluated using a measuring system for *in vitro* myostatin transcriptional activity. Luciferase activity (luciferase activity/β-galactosidase activity) from the cell treated with ASO at each concentration is shown. IC50 was 87.4 nM. Three independent experiments were conducted and the results were summarized. *P<0.05, ***P<0.001.
[Fig. 20]
   MSTN_Ex3_7-2b was administered to human myoblast cells at the final concentration of 0, 1, 5, 10, 25, 50 or 100 nM and a cell proliferation test was conducted with CCK-8. The results at day 3 of administration are shown. Three independent experiments were conducted and the results were summarized. *P<0.05, ***P<0.001.
[Fig. 21]
   MSTN_Ex3_7-2b was administered to human rhabdomyosarcoma cells (CRL-2061) at the final concentration of 0, 1, 5, 10, 25, 50 or 100 nM and a cell proliferation test was conducted with CCK-8. The results at day 3 of administration are shown. Three independent experiments were conducted and the results were summarized. *P<0.05, **P<0.01,
   ***P<0.001.

### BEST MODES FOR CARRYING OUT THE INVENTION

Hereinbelow, embodiments of the present invention will be described in detail.

The present invention provides an antisense oligonucleotide of 15-30 bases or a salt or a solvate thereof, wherein the antisense oligonucleotide has a nucleotide sequence complementary to a target sequence in exon 3 of the myostatin gene and is capable of allowing the expression of a splicing variant of myostatin. The present inventors previously showed that expression of myostatin-b (MSTN-b), a myostatin (MSTN) isoform, in cultured cells decreased myostatin signaling (WO2020/179571). This result suggests that myostatin-b inhibits myostatin and promotes myogenesis. The nucleotide sequence of MSTN-b is shown in SEQ ID NO: 68. Myostatin is composed of signal peptide (nucleotide No. 1 to No. 18), prodomain (nucleotide No. 19 to No. 266) and mature myostatin (nucleotide No. 267 to No. 375) in this order from N terminus. Nucleotide sequences of myostatin and myostatin-b are identical in exon 1 and exon 2, with commonality of up to amino acid No. 249. Since myostatin-b is different in the nucleotide sequence of exon 3, the 250^{th} amino acid from N terminus is asparagine (N), the 251^{st} amino acid is valine (V) and the 252^{nd} position is a stop codon (*). For this reason, myostatin-b does not have the domain of mature myostatin. The amino acid sequence of myostatin-b protein is shown in SEQ ID NO: 69. The amino acid sequence of myostatin is shown in SEQ ID NO: 70. With the antisense oligonucleotide of the present invention, it is possible to switch the splicing of the myostatin gene from production of myostatin to production of myostatin-b. The antisense oligonucleotide of the present invention is capable of decreasing myostatin and increasing myostatin-b in myostatin-expressing cells at the protein level. Further, the antisense oligonucleotide of the present invention is also capable of decreasing myostatin signaling.

The nucleotide sequence of exon 3 of the human myostatin gene is shown in SEQ ID NO: 1. In the present invention, when the nucleotide sequence of exon 3 of the human myostatin gene is the nucleotide sequence as shown in SEQ ID NO: 1, a target sequence in exon 3 of the myostatin gene may be suitably located within the region of nucleotide Nos. 10-33 of the nucleotide sequence as shown in SEQ ID NO: 1. The nucleotide sequence of the region of nucleotide Nos. 10-33 of the nucleotide sequence as shown in SEQ ID NO: 1 is shown in SEQ ID NO: 2. As used herein, "target sequence" refers to such a sequence that by means of an ASO having a sequence complementary to whole or part of said sequence, the production of a splicing variant of myostatin is promoted. The ASO that promotes the production of a splicing variant of myostatin may conveniently comprise a sequence complementary to a sequence consisting of at least 15 consecutive nucleotides in the nucleotide sequence as shown in SEQ ID NO: 2.

Further, in the present invention, the nucleotide sequence of the antisense oligonucleotide may conveniently comprise a sequence consisting of at least 15 consecutive nucleotides in any one of the sequences as shown in SEQ ID NO: 24, 30, 31, 27, 32, 33 or 28 (wherein "t" may be "u", and "u" may be "t"). The nucleotide sequences as shown in SEQ ID NOS: 24, 30, 31, 27, 32, 33 and 28 have in common a sequence complementary to the sequence of the region of nucleotide Nos. 7-18 of the nucleotide sequence as shown in SEQ ID NO: 2. The nucleotide sequences as shown in SEQ ID NOS: 61-67 are examples of sequences wherein "t" is changed to "u".

The antisense oligonucleotide may have 18 bases, and the nucleotide sequence of the antisense oligonucleotide may be any one of the sequences as shown in SEQ ID NO: 24, 30, 31, 27, 32, 33 or 28 (wherein "t" may be "u", and "u" may be "t").

Nucleotides constituting the antisense oligonucleotide may be either natural DNA, natural RNA, or modified DNA or RNA. Preferably, at least one of the nucleotides is a modified nucleotide.

Examples of modified nucleotides include those in which a sugar is modified (e.g., the hydroxy group at 2'-position of D-ribofuranose is modified as in the case where D-ribofuranose is 2'-O-alkylated or 2'-O,4'-C-alkylenated), those in which a phosphodiester bond is modified (e.g., thioated), those in which a base is modified, combinations of the above-described nucleotides, and so forth. Antisense oligonucleotides in which at least one D-ribofuranose constituting the oligonucleotides is 2'-O-alkylated or 2'-O,4'-C-alkylenated have high RNA binding strength and high resistance to nuclease. Thus, they are expected to produce higher therapeutic effect than natural nucleotides (i.e. oligo DNA or oligo RNA). Further, oligonucleotides in which at least one phosphodiester bond constituting the oligonucleotides is thioated also have high resistance to nuclease and, thus, are expected to produce higher therapeutic effect than natural nucleotides (i.e. oligo DNA or oligo RNA). Oligonucleotides comprising both the modified sugar and the modified phosphate as described above have even higher resistance to nuclease and, thus, are expected to produce even higher therapeutic effect.

With respect to the antisense oligonucleotide, examples of modified sugars include, but are not limited to, D-ribofuranose as 2'-O-alkylated (e.g. 2'-O-methylated, 2'-O-aminoethylated, 2'-O-propylated, 2'-O-allylated, 2'-O-methoxyethylated, 2'-O-butylated, 2'-O-pentylated, or 2'-O-propargylated); D-ribofuranose as 2'-O,4'-C-alkylenated (e.g. 2'-O,4'-C-ethylenated, 2'-O,4'-C-methylenated, 2'-O,4'-C-propylenated, 2'-O,4'-C-tetramethylenated, or 2'-O,4'-C-pentamethylenated); 3'-deoxy-3'-amino-2'-deoxy-D-ribofuranose, 3'-deoxy-3'-amino-2'-deoxy-2'-fluoro-D-ribofuranose, etc.

With respect to the antisense oligonucleotide, examples of the modification of phosphodiester bond include, but are not limited to, phosphorothioate bond, methylphosphonate bond, methylthiophosphonate bond, phosphorodithioate bond and phosphoramidate bond.

With respect to the antisense oligonucleotide, examples of modified bases include, but are not limited to, cytosine as 5-methylated, 5-fluorinated, 5-brominated, 5-iodinated or N4-methylated; thymidine as 5-demethylated (uracil), 5-fluorinated, 5-brominated or 5-iodinated; adenine as N6-methylated or 8-brominated; guanine as N2-methylated or 8-brominated; and uridine as pseudouridinated or 1-methylpseudouridinted.

The antisense oligonucleotide of the present invention may be used in the form of a salt. When the antisense oligonucleotide of the present invention is used in a pharmaceutical drug, salts may suitably be pharmaceutically acceptable salts. Examples of such salts include, but are not limited to, alkaline metal salts such as sodium salts, potassium salts or lithium salts; alkaline earth metal salts such as calcium salts or magnesium salts; metal salts such as aluminum salts, iron salts, zinc salts, copper salts, nickel salts or cobalt salts; amine salts including inorganic salts such as ammonium salts and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts or tris(hydroxymethyl)aminomethane salts; inorganic acid salts including hydrohalogenic acid salts such as hydrofluorides, hydrochlorides, hydrobromides or hydroiodides, as well as nitrates, perchlorates, sulfates or phosphates; organic acid salts including lower alkane sulfonic acid salts such as methanesulfonates, trifluoromethanesulfonates or ethanesulfonates, arylsulfonic acid salts such as benzenesulfonates or p-toluenesulfonates, as well as acetates, malates, fumarates, succinates, citrates, tartrates, oxalates or maleates; and amino acid salts such as glycine salts, lysine salts, arginine salts, ornithine salts, glutamic acid salts or aspartic acid salts. These salts may be prepared by known methods.

The antisense oligonucleotide sometimes occur as a solvate (e.g., hydrate). The antisense oligonucleotide may be such a solvate.

Further, the antisense oligonucleotide may be administered in the form of a prodrug. Examples of such prodrug include, but are not limited to, amides, esters, carbamates, carbonates, ureides and phosphates. These prodrugs may be prepared by known methods.

As methods of synthesizing the antisense oligonucleotide, conventional methods may be used. Conventional methods include, but are not limited to, synthesis methods using genetic engineering techniques and chemical synthesis methods. Genetic engineering techniques include, but are not limited to, *in vitro* transcription synthesis method, method using vectors, and method using PCR cassettes. The vector is not particularly limited. Nonviral vectors such as plasmids, virus vectors, etc. may be used. The chemical synthesis method is not particularly limited. For example, the phosphoramidite method, the H-phosphonate method, or the like may be enumerated. For the chemical synthesis method, a commercially available automated nucleic acid synthesizer, for example, may be used. Generally, amidites are used in the chemical synthesis method. The amidite is not particularly limited. In Examples described later, antisense oligonucleotides were synthesized by the phosphoramidite method using ENA-2CE phosphoramidite and 2'OMe-2-CE phosphoramidite.

As regards phosphoramidite reagents to be used, natural nucleosides and 2'-O-methylnucleosides (i.e., 2'-O-methylguanosine, 2'-O-methyladenosine, 2'-O-methylcytosine and 2'-O-methyluridine) are commercially available. As regards 2'-O-alkylguanosine, - alkyladenosine, -alkylcytosine and -alkyluridine in which the carbon number of the alkyl group is 2-6, the following methods may be employed.

2'-O-aminoethylguanosine, -aminoethyladenosine, -aminoethylcytosine and -aminoethyluridine may be synthesized as previously described (Blommers et al., Biochemistry (1998), 37, 17714-17725).

2'-O-propylguanosine, -propyladenosine, -propylcytosine and -propyluridine may be synthesized as previously described (Lesnik, E.A. et al., Biochemistry (1993), 32, 7832-7838).

For the synthesis of 2'-O-allylguanosine, -allyladenosine, -allylcytosine and -allyluridine, commercially available reagents may be used.

2'-O-methoxyethylguanosine, -methoxyethyladenosine, -methoxyethylcytosine and -methoxyethyluridine may be synthesized as previously described (US Patent No. 6261840 or Martin, P. Helv. Chim. Acta. (1995) 78, 486-504).

2'-O-butylguanosine, -butyladenosine, -butylcytosine and -butyluridine may be synthesized as previously described (Lesnik, E.A. et al., Biochemistry (1993), 32, 7832-7838).

2'-O-pentylguanosine, -pentyladenosine, -pentylcytosine and -pentyluridine may be synthesized as previously described (Lesnik, E.A. et al., Biochemistry (1993), 32, 7832-7838).

For the synthesis of 2'-O-propargylguanosine, -propargyladenosine, -propargylcytosine and -propargyluridine, commercially available reagents may be used.

2'-O,4'-C-methyleneguanosine, 2'-O,4'-C-methyleneadenosine, 5-methylcytosine and 5-methylthymidine may be prepared according to the method described in WO99/14226; and 2'-O,4'-C-alkyleneguanosine, 2'-O,4'-C-alkyleneadenosine, 5-methylcytosine and 5-methylthymidine in which the carbon number of the alkylene group is 2-5 may be prepared according to the method described in WO00/47599.

An antisense oligonucleotide with phosphorothioate bonds can be synthesized by coupling phosphoramidite reagents and then reacting sulfur, tetraethylthiuram disulfide (TETD; Applied Biosystems), Beaucage reagent (Glen Research) or a reagent such as xanthan hydride (Tetrahedron Letters, 32, 3005 (1991); J. Am. Chem. Soc. 112, 1253 (1990); PCT/WO98/54198).

As controlled pore glass (CPG) to be used in a DNA synthesizer, 2'-O-methylnucleoside-bound CPG is commercially available. As regards 2'-O,4'-C-methyleneguanosine, 2'-O,4'-C-methyleneadenosine, 5-methylcytosine and 5-methylthymidine, they may be prepared according to the method described in WO99/14226; and as regards 2'-O,4'-C-alkyleneguanosine, 2'-O,4'-C-alkyleneadenosine, 5-methylcytosine and 5-methylthymidine in which the carbon number of the alkylene group is 2-5, they may be prepared according to the method described in WO00/47599. The thus prepared nucleosides may then be bound to CPG as previously described (Oligonucleotide Synthesis, Edited by M. J. Gait, Oxford University Press, 1984). By using the modified CPG (as disclosed in Example 12b of Japanese Unexamined Patent Publication No. Hei7-87982), an oligonucleotide in which a 2-hydroxyethylphosphate group is bound at the 3' end can be synthesized. If 3'-amino-Modifier C3 CPG, 3'-amino-Modifier C7 CPG or Glyceryl CPG (Glen Research) or 3'-specer C3 SynBase CPG 1000 or 3'-specer C9 SynBase CPG 1000 (Link Technologies) is used, an oligonucleotide in which a hydroxyalkylphosphate group or aminoalkylphosphate group is bound at the 3' end can be synthesized.

The antisense oligonucleotide of the present invention may be used in a pharmaceutical drug. When used in a pharmaceutical drug, the antisense oligonucleotide may be in the form of a pharmaceutically acceptable salt, solvate or prodrug. Therefore, the present invention provides a pharmaceutical drug comprising the above-described antisense oligonucleotide, or a pharmaceutically acceptable salt or solvate thereof. The pharmaceutical drug may be for preventing and/or treating a pathological condition and/or a disease in which myostatin is involved. Examples of the condition and/or disease in which myostatin is involved include, but are not limited to, muscular atrophy (e.g., muscular dystrophy, myopathy, spinal muscular atrophy, sarcopenia and disuse muscle atrophy), as well as conditions and/or diseases in which a therapeutic effect is gained through muscle mass recovery (e.g., cancer cachexia, diabetes, cardiovascular diseases such as cardiac failure or arteriosclerosis, renal diseases such as chronic renal failure, and bone diseases such as inflammatory arthritis). Since myostatin inhibition leads to an increase in skeletal muscle mass, it is believed that myostatin inhibition can be used for treatment of all diseases that present with muscular atrophy regardless of its cause. Increased skeletal muscle mass contributes to increasing the amount of exercise and hence improving metabolism in the whole body. Further, myostatin inhibition can be expected to affect the cardiac muscle to thereby recover its function. In addition, myostatin inhibition is also expected to have the following effects: affecting osteoclasts to thereby inhibit bone resorption; activating the homeostasis capacity of vascular endothelial cells; inducing apoptosis; increasing insulin sensitivity; and so forth. The present invention also provides a method of preventing and/or treating a pathological condition and/or a disease in which myostatin is involved, comprising administering to a subject an effective amount of the above-described antisense oligonucleotide or a salt or a solvate thereof. Further, the present invention also provides the above-described antisense oligonucleotide or a salt or a solvate thereof, for use in a method of preventing and/or treating a pathological condition and/or a disease in which myostatin is involved. Still further, the present invention provides use of the above-described antisense oligonucleotide or a salt or a solvate thereof, for preventing and/or treating a pathological condition and/or a disease in which myostatin is involved.

The antisense oligonucleotide of the present invention or a pharmaceutically acceptable salt, solvate or prodrug thereof (hereinafter, referred to as "active ingredient") may be administered, either alone or together with pharmacologically acceptable carriers, diluents or excipients in appropriate forms of preparations, to mammals (e.g., human, rabbit, dog, cat, rat, mouse, etc.) orally or parenterally. Dose levels may vary depending on the subject to be treated, the target disease, symptoms, administration route, and so on. For example, in the case of use for prevention/treatment of an muscle wasting disease (such as muscular dystrophy), typically about 0.1 to 50 mg/kg body weight, preferably about 0.5 mg /kg body weight, may be administered as a dosage unit in terms of active ingredient at a frequency of about once a week or a month, preferably at a frequency of about once a year, either orally or by intramuscular, subcutaneous or intravenous injection (preferably administered consecutively or on alternate days). In cases of other parenteral administration and oral administration, similar dose levels may be used. If symptoms are very severe, the dosage may be increased accordingly. For other diseases, dose levels may be appropriately increased or decreased with reference to the above-described levels.

Preparations for oral administration include solid or liquid preparations such as tablets (including sugar-coated tablets and film-coated tablets), pills, granules, powders, capsules (including soft capsules), syrups, emulsions and suspensions. These preparations may be prepared according to conventional methods and may contain those carriers, diluents or excipients which are conventionally used in the pharmaceutical formulation procedure. For example, lactose, starch, sucrose, magnesium stearate and the like may be used as carriers or excipients for tablets.

Preparations for parenteral administration include, for example, injections and suppositories. Injections include intravenous injections, subcutaneous injections, intradermal injections, muscle injections, instilment injections, etc. Such injections may be prepared by conventional methods, i.e., by dissolving, suspending or emulsifying the active ingredient in an aseptic, aqueous or oily liquid that is conventionally used in injections. Examples of aqueous liquids for injection include physiological saline and isotonic solutions containing glucose and other auxiliary agents. They may be used in combination with a suitable auxiliary solubilizer such as alcohol (e.g. ethanol), polyalcohol (e.g. propylene glycol, polyethylene glycol), nonionic surfactant [e.g. Polysorbate 80, HCO-50 (polyoxyethylene (50 mol) adduct of hydrogenated castor oil)], etc. Examples of oily liquids for injection include sesame oil and soybean oil. They may be used in combination with an auxiliary solubilizer such as benzyl benzoate, benzyl alcohol, etc. Usually, the prepared injections are filled in appropriate ampoules. Suppositories for administration into the rectum may be prepared by mixing the active ingredient with a conventional suppository base.

It is convenient to formulate the above-described pharmaceutical preparations for oral or parenteral administration into unit dosage forms that would give an appropriate dose of the active ingredient. Examples of such unit dosage forms include tablets, pills, capsules, injections (ampoules), and suppositories. Typically 0.1 to 1000 mg of the active ingredient is preferably contained in each unit dosage form.

The antisense oligonucleotide of the present invention may also be used in food or feed. For example, the antisense oligonucleotide of the present invention may be used as an additive to food or feed, or may be used as a supplement for human or animals. The antisense oligonucleotide of the present invention may be in the form of a salt, a solvate or a prodrug that are acceptable as a food or feed ingredient. Therefore, the present invention provides a food comprising the antisense oligonucleotide or a salt or a solvate thereof that are acceptable as a food ingredient. The present invention also provides a feed comprising the antisense oligonucleotide or a salt or a solvate thereof that are acceptable as a feed ingredient. As one example of the salt, solvate or prodrug that are acceptable as a food or feed ingredient, a pharmaceutically acceptable salt, solvate or prodrug may be given. For details, reference should be had to the foregoing description.

It is possible to promote myocyte proliferation and/or hypertrophy using the antisense oligonucleotide of the present invention. Myocytes are contractile cells that form muscle tissues in human and animals and they include skeletal muscle cell, smooth muscle cell, cardiomyocyte, etc. Myostatin inhibition by the antisense oligonucleotide of the present invention is effective in myoblasts and induces the promotion of myoblast proliferation and differentiation to be capable of eventually causing the proliferation and hypertrophy of myocytes. Myocytes also include precursor cells such as myoblasts. The antisense oligonucleotide of the present invention may be in the form of a salt, a solvate or a prodrug. As one example of salt, solvate or prodrug, a pharmaceutically acceptable salt, solvate or prodrug may be given. For details, reference should be had to the foregoing description. Animals may be those which are expressing myostatin, as exemplified by domestic animals used for work or food, or fishes under culture, specifically by mammals such as cat, dog, sheep, pig or cattle, poultry such as chicken or turkey, and fishes such as salmon, trout, cod fish, tuna or yellowtail. Therefore, the present invention provides an agent for promoting myocyte proliferation and/or hypertrophy, comprising the above-described antisense oligonucleotide or a salt or a solvate thereof. The present invention also provides a method of promoting myocyte proliferation and/or hypertrophy, comprising administering to a subject an effective amount of the antisense oligonucleotide or a salt or a solvate thereof. The present invention also provides the above-described antisense oligonucleotide or a salt or a solvate thereof, for use in a method of promoting myocyte proliferation and/or hypertrophy. The present invention also provides use of the above-described antisense oligonucleotide or a salt or a solvate thereof, for promoting myocyte proliferation and/or hypertrophy. The subject may be a human or an animal. Animals are as described above. The dosage form, dose level, administration route, administration frequency, etc. of the agent may be determined based on the above-described pharmaceutical drug, and they may be changed appropriately so that the desired effect can be obtained.

Further, the antisense oligonucleotide of the present invention may be used for increasing muscle mass or for inhibiting muscle weakness in human or animals. The antisense oligonucleotide of the present invention may be in the form of a salt, a solvate or a prodrug. As one example of salt, solvate or prodrug, a pharmaceutically acceptable salt, solvate or prodrug may be given. For details, reference should be had to the foregoing description. Animals may be those which are expressing myostatin, as exemplified by domestic animals used for work or food, or fishes under culture, specifically by mammals such as cat, dog, sheep, pig or cattle, poultry such as chicken or turkey, and fishes such as salmon, trout, cod fish, tuna or yellowtail. Therefore, the present invention provides an agent for increasing muscle mass and/or inhibiting muscle weakness, comprising the antisense oligonucleotide or a salt or a solvate thereof. The present invention also provides a method of increasing muscle mass and/or inhibiting muscle weakness, comprising administering to a subject an effective amount of the antisense oligonucleotide or a salt or a solvate thereof. The present invention also provides the antisense oligonucleotide or a salt or a solvate thereof, for use in a method of increasing muscle mass and/or inhibiting muscle weakness. The present invention also provides use of the antisense oligonucleotide or a salt or a solvate thereof, for increasing muscle mass and/or for inhibiting muscle weakness. The subject may be a human or an animal. Animals are as described above. The dosage form, dose level, administration route, administration frequency, etc. of the agent may be determined based on the above-described pharmaceutical drug, and they may be changed appropriately so that the desired effect can be obtained.

The food to which the antisense oligonucleotide of the present invention or a salt, a solvate or a prodrug thereof that are acceptable as a food ingredient is added may be any food such as plant-derived foods, animal-derived foods, fungus-derived foods, fresh foods, processed foods, nonessential tasty foods, ingredients to be cooked or seasoned, drinks, health foods, space foods, pet foods, etc.

The following may be added to the food of the present invention: common ingredients such as protein, fat, carbohydrate and sodium; minerals such as potassium, calcium, magnesium and phosphorus; trace elements such as iron, zinc, copper, selenium and chromium; vitamins such as vitamin A, β-carotene, vitamin B₁, vitamin B₂, vitamin B₆, vitamin B₁₂, vitamin C, niacin, folic acid, vitamin D₃, vitamin E, biotin and pantothenic acid; and other substances such as coenzyme Q10, α-lipoic acid, galacto-oligosaccharide, dietary fiber, excipients (such as water, carboxymethyl cellulose or lactose), sweeteners, flavoring agents (such as malic acid, citric acid or amino acids), and fragrances. When the food of the present invention is provided as a liquid food, water, physiological saline, soup, milk, fruit juice or the like can be used as a liquid in which the food ingredients are dispersed or dissolved. The food of the present invention may be formulated into such forms as powder, granules, tablets or liquid preparations. In order to allow easy intake by patients or elderly persons, the food of the present invention preferably assumes the form of a gel-like product such as jelly.

The feed to which the antisense oligonucleotide of the present invention or a salt, a solvate or a prodrug thereof acceptable as a feed ingredient is added may be any feed such as a single feed (e.g., cereal, vegetable oil meal, rice bran, food manufacturer's by-products (lees), animal feed, etc.) or a mixed feed comprising multiple feed materials and feed additives.

The feed of the present invention may comprise the following additives: antioxidants (such as ethoxyquin, dibutylhydroxytoluene, butylhydroxyanisole, etc.), mold inhibitors (such as propionic acid, calcium propionate, sodium propionate, etc.), thickeners (such as sodium alginate, sodium caseinate, sodium carboxymethylcellulose, propylene glycol, sodium polyacrylate, etc.), emulsifiers (such as glycerin fatty acid ester, sucrose fatty acid ester, sorbitan fatty acid ester, polyoxyethylene sorbitan fatty acid ester, polyoxyethylene glycerin fatty acid ester, etc.), adjusters (such as formic acid), amino acids and the like (such as aminoacetic acid, DL-alanine, L-arginine, L-lysine hydrochloride, L-carnitine, guanidinoacetic acid, sodium L-glutamate, taurine, 2-deamino-2-hydroxymethionine, DL-tryptophan, L-tryptophan, L-threonine, L-valine, DL-methionine, L-lysine sulphate, etc.), vitamins (such as L-ascorbic acid, calcium L-ascorbate, sodium L-ascorbate, sodium-calcium-L-ascorbic acid-2-phosphate ester, magnesium L-ascorbic acid-2-phosphate ester, acetomenaphthone, inositol, dibenzoylthiamine hydrochloride, ergocalciferol, choline chloride, thiamine hydrochloride, pyridoxine hydrochloride, β-carotene, cholecalciferol, DL-α-tocopherol acetate, cyanocobalamin, thiamine mononitrate, nicotinic acid, nicotinamide, p-aminobenzoic acid, calcium D-pantothenate, calcium DL-pantothenate, d-biotin, vitamin A powder, vitamin A oil, vitamin D powder, vitamin D3 oil, vitamin E powder, 25-hydroxycholecalciferol, menadione dimethylpyrimidinol bisulfite, menadione sodium bisulfite, folic acid, riboflavin, riboflavin butyrate ester, etc.), minerals (such as potassium chloride, ferric citrate, calcium gluconate, iron and sodium succinate citrate, magnesium oxide, aluminum hydroxide, zinc carbonate, cobalt carbonate, sodium bicarbonate, magnesium carbonate, manganese carbonate, zinc 2-deamino-2-hydroxymethionine, ferrous DL-threonate, calcium lactate, ferrous fumarate, zinc peptide, iron peptide, copper peptide, manganese peptide, potassium iodide, potassium iodate, calcium iodate, zinc sulfate (dry), zinc sulfate (crystal), zinc methionine sulfate, sodium sulfate (dry), magnesium sulfate (dry), magnesium sulfate (crystal), cobalt sulfate (dry), cobalt sulfate (crystal), ferrous sulfate (dry), copper sulfate (dry), copper sulfate (crystal), manganese sulfate, dipotassium hydrogen phosphate (dry), disodium hydrogen phosphate (dry), potassium dihydrogen phosphate (dry), sodium dihydrogen phosphate (dry), sodium dihydrogen phosphate (crystal), etc.), pigments, synthetic antimicrobials, antibiotics, odorizers, flavoring agents, enzymes, probiotics, organic acids, and so forth.

Intake of the food or feed of the present invention may be performed in such an amount, frequency and a period of intake that the desired effect (e.g., promotion of myogenesis) can be confirmed.

The antisense oligonucleotide of the present invention is capable of switching the splicing of the myostatin gene from production of myostatin to production of a slicing variant thereof. Therefore, the present invention provides an agent for switching the splicing of the myostatin gene from production of myostatin to production of a splicing variant thereof, comprising the antisense oligonucleotide of the present invention. The agent of the present invention may be used as a pharmaceutical drug for human or animals; as an additive to food or feed or a supplement; as a growth promoting agent for animals; or as a reagent for experiments. The antisense oligonucleotide of the present invention may be in the form of a salt, a solvate or a prodrug. As one example of salt, solvate or prodrug, a pharmaceutically acceptable salt, solvate or prodrug may be given. For details, reference should be had to the foregoing description. Further, the present invention provides a method of switching the splicing of the myostatin gene from production of myostatin to production of a splicing variant thereof, comprising administering to a subject an effective amount of the above-described antisense oligonucleotide or a salt or a solvate thereof. The present invention also provides the above-described antisense oligonucleotide or a salt or a solvate thereof, for use in a method of switching the splicing of the myostatin gene from production of myostatin to production of a splicing variant thereof. The present invention also provides use of the above-described antisense oligonucleotide or a salt or a solvate thereof, for switching the splicing of the myostatin gene from production of myostatin to production of a splicing variant thereof.

The antisense oligonucleotide of the present invention is capable of decreasing myostatin signaling. Therefore, the present invention provides an agent for decreasing myostatin signaling, comprising the antisense oligonucleotide of the present invention. The agent for decreasing myostatin signaling of the present invention may be used as a pharmaceutical drug for human or animals; as an additive to food or feed or a supplement; as a growth promoting agent for animals; or as a reagent for experiments. The antisense oligonucleotide of the present invention may be in the form of a salt, a solvate or a prodrug. As one example of salt, solvate or prodrug, a pharmaceutically acceptable salt, solvate or prodrug may be given. For details, reference should be had to the foregoing description. Further, the present invention also provides a method of decreasing myostatin signaling, comprising administering to a subject an effective amount of the above-described antisense oligonucleotide or a salt or a solvate thereof. The present invention also provides the above-described antisense oligonucleotide or a salt or a solvate thereof, for use in a method of decreasing myostatin signaling. The present invention also provides use of the above-described antisense oligonucleotide or a salt or a solvate thereof, for decreasing myostatin signaling. Since it is considered that inhibition of myostatin signaling would be effective for suppression of diabetes or inhibition of its progression, the above-described antisense oligonucleotide or a salt or a solvate thereof could be effective for prevention and/or treatment of diabetes. Therefore, the present invention also provides an agent for preventing and/or treating diabetes, comprising the above-described antisense oligonucleotide or a salt or a solvate thereof.

When the antisense oligonucleotide of the present invention is used as a reagent for experiments, myostatin-expressing cells, tissues or organs are treated with the antisense oligonucleotide of the present invention or a salt or a solvate thereof, whereby the splicing of the myostatin gene is switched from production of myostatin to production of a splicing variant thereof. As a result, expression of a myostatin isoform can be induced. With this isoform, myostatin signaling can be inhibited. The antisense oligonucleotide of the present invention or a salt or a solvate thereof may be used in an amount effective for an exertion of the desired function. The myostatin-expressing cells may be exemplified by myocytes, myosarcoma cells, and cancer cells of digestive organs, lung, esophagus, etc. In addition to naturally occurring cells, myostatin gene-transfected recombinant cells may also be employed. The myostatin-expressing tissues and organs may be exemplified by skeletal muscles, cardiac muscles, blood vessels, kidney, digestive organs, uterus, liver, spleen, lung, etc. The switching of the splicing of the myostatin gene from production of myostatin to production of a splicing variant thereof may be examined, for example, by analyzing the mRNA of a splicing variant of myostatin in samples by RT-PCR; or by detecting a protein translated from a splicing variant of myostatin in samples either by Western blotting or by mass spectrometry.

The antisense oligonucleotide of the present invention is capable of inhibiting the growth of cancer cells. The cancer cells may be exemplified by rhabdomyosarcoma cells, liver cancer cells, cervical cancer cells, alveolar epithelial adenocarcinoma cells, etc. Therefore, the present invention provides an anticancer agent comprising the above-described antisense oligonucleotide. Indications for the anticancer agent of the present invention include, but are not limited to, sarcoma, liver cancer, cervical cancer, and lung cancer. The anticancer agent of the present invention may be used as a pharmaceutical drug for human or animals. The antisense oligonucleotide of the present invention may be in the form of a salt, a solvate or a prodrug. As one example of salt, solvate or prodrug, a pharmaceutically acceptable salt, solvate or prodrug may be given. For details, reference should be had to the foregoing description. Further, the present invention provides a method of preventing and/or treating cancer, comprising administering to a subject an effective amount of the above-described antisense oligonucleotide or a salt or a solvate thereof. The present invention also provides the above-described antisense oligonucleotide or a salt or a solvate thereof, for use in a method of preventing and/or treating cancer. The present invention also provides use of the above-described antisense oligonucleotide or a salt or a solvate thereof, for preventing and/or treating cancer. Prevention of cancer is a concept encompassing recurrence of cancer.

### EXAMPLES

Hereinbelow, the present invention will be described in detail with reference to the following Examples. However, the present invention is not limited to these Examples.

### [Examples 1-54] Synthesis of Antisense Oligonucleotides (ASOs)

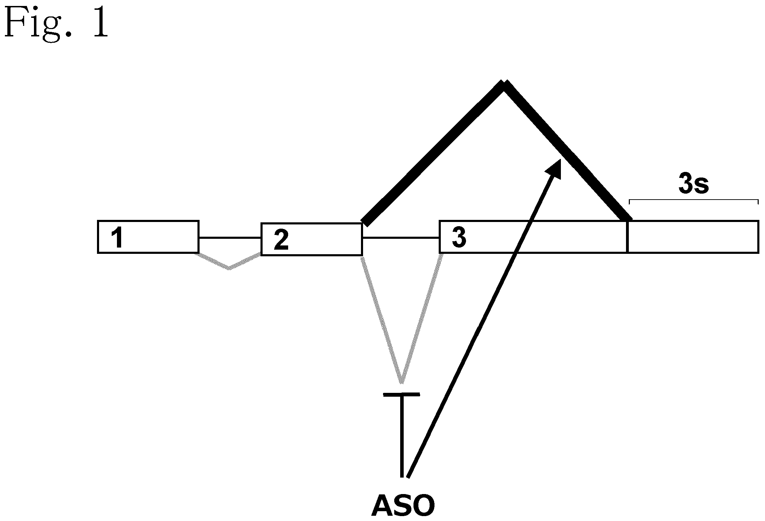

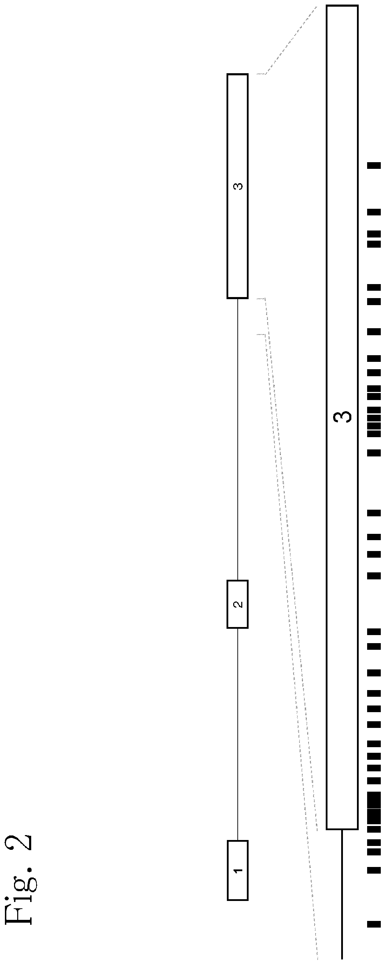

In order to search for ASOs which would promote alternative splicing for the production of MSTN-b (see Fig. 1), the present inventors synthesized the antisense oligonucleotides (ASOs) as shown in Table 1. Recognition sites of ASOs complementary to MSTN pre-mRNA are shown in Fig. 2. Modified nucleic acid ENAs (2'-O,4'-C-Ethylene-bridged Nucleic Acids) were introduced into A (adenine), G (guanine), C (cytosine) and T (thymine) in ASO sequences to provide improved affinity and stability.

### Synthesis of gaaCaaTCagTaaTaTCa (MSTN _Ex3 _BP) (Example 1)

Synthesis was performed with an automated nucleic acid synthesizer (DNA/RNA synthesizer model NTS H-6: Nihon Techno Service Co., Ltd.) at 1 µmol scale. Concentrations of solvents, reagents and phosphoramidites in each synthesis cycle were the same as those concentrations used in natural oligonucleotide synthesis. Reagents and 2'-O-methylnucleoside phosphoramidites (for adenosine: Product No. 10-3100-10; for guanosine: Product No. 10-3121-10) were available from Glen Research. Solvents used were products from Wako Pure Chemical Industries. Non-natural phosphoramidites used were the following compounds disclosed in Japanese Unexamined Patent Publication No. 2000-297097 at Example 22 (5'-O-dimethoxytrityl-2'-O,4'-C-ethylene-4-N-benzoyl-5-methylcytidine-3'-O-(2-cyanoethyl N,N-diisopropyl)phosphoramidite) and Example 9 (5'-O-dimethoxytrityl-2'-O,4'-C-ethylene-5-methyluridine-3'-O-(2-cyanoethyl N,N-diisopropyl)phosphoramidite). As a solid carrier, universal controlled pore glass (CPG) (Product No. 25-5040; Glen Research) was used. Thus, the titled compound was synthesized. It should be noted here that 15 minutes was set as the time required for condensation of amidites.

Protected oligonucleotide analogs with the sequence of interest were thermally treated with thick aqueous ammonia (55°C, 8 hrs) to thereby cut out oligomers from the support and, at the same time, remove the protective group cyanoethyl on phosphorus atoms and the protective group on nucleobases. The resultant ammonia solution was transferred into Glen-Pak DNA Purification Cartridge (Product No. 60-5100; Glen Research), in which DMT groups were removed according to the protocol recommended by Glen Research. The recovered solution was evaporated under reduced pressure; the residue was purified by reversed phase HPLC [(LC-2a from Shimadzu Corporation; column (Triart C18 from YMC (10x150 mm)); Solution A: 0.1M triethylamine acetate aqueous solution (TEAA), pH 7.0; Solution B: acetonitrile, B%: from 10% to 25% (30 min, linear gradient); 50°C; 4.7 mL/min; 280 nm]. After distilling off the solvent, the residue was dissolved in 10 mM NaOH solution, and pure water substitution was performed by ultrafiltration using a Microsep centrifugal filtration device (Product No. MCP003C; Nippon Pall). After lyophilization, a compound of interest was obtained.

The subject compound was identified by negative-ion MALDI-TOFMS (theoretical: 6440.35, found: 6433.59).

The nucleotide sequence of the subject compound is a sequence complementary to nucleotide Nos. 5058-5075 of Homo sapiens myostatin (MSTN), RefSeqGene (LRG_200) on chromosome 2 (Gene Bank accession No. NG_009800.1).

### Synthesis of aaaCggaTTCTgTTTgaa (MSTN Ex3U_SS) (Example 2)

The compound of Example 2 with the sequence of interest was synthesized in the same manner as described for the compound of Example 1.

The subject compound was identified by negative-ion MALDI-TOFMS (theoretical: 6476.33, found: 6469.82).

The nucleotide sequence of the subject compound is a sequence complementary to nucleotide Nos. 5083-5100 of Homo sapiens myostatin (MSTN), RefSeqGene (LRG_200) on chromosome 2 (Gene Bank accession No. NG_009800.1).

### Synthesis of TCaTCaCagTCaagaCCa (MSTN Ex3_Fox1) (Example 3)

The compound of Example 3 with the sequence of interest was synthesized in the same manner as described for the compound of Example 1.

The subject compound was identified by negative-ion MALDI-TOFMS (theoretical: 6443.39, found: 6436.58).

The nucleotide sequence of the subject compound is a sequence complementary to nucleotide Nos. 5184-5165 of Homo sapiens myostatin (MSTN), RefSeqGene (LRG_200) on chromosome 2 (Gene Bank accession No. NG_009800.1).

### Synthesis of CCTgCTgaaCCTCTgggg (MSTN Ex3__-LESE12SF2) (Example 4)

The compound of Example 4 with the sequence of interest was synthesized in the same manner as described for the compound of Example 1.

The subject compound was identified by negative-ion MALDI-TOFMS (theoretical: 6510.38, found: 6503.83).

The nucleotide sequence of the subject compound is a sequence complementary to nucleotide Nos. 5337-5354 of Homo sapiens myostatin (MSTN), RefSeqGene (LRG_200) on chromosome 2 (Gene Bank accession No. NG_009800.1).

### Synthesis of aTTgTTgaggggaaaaCC (MSTN Ex3_LESE3) (Example 5)

The compound of Example 5 with the sequence of interest was synthesized in the same manner as described for the compound of Example 1.

The subject compound was identified by negative-ion MALDI-TOFMS (theoretical: 6502.33, found: 6495.71).

The nucleotide sequence of the subject compound is a sequence complementary to nucleotide Nos. 5512-5529 of Homo sapiens myostatin (MSTN), RefSeqGene (LRG_200) on chromosome 2 (Gene Bank accession No. NG_009800.1).

### Synthesis of TaTagCCTgTggTaCTTa (MSTN _Ex3_LESE4) (Example 6)

The compound of Example 6 with the sequence of interest was synthesized in the same manner as described for the compound of Example 1.

The subject compound was identified by negative-ion MALDI-TOFMS (theoretical: 6481.33, found: 6474.94).

The nucleotide sequence of the subject compound is a sequence complementary to nucleotide Nos. 5546-5563 of Homo sapiens myostatin (MSTN), RefSeqGene (LRG_200) on chromosome 2 (Gene Bank accession No. NG_009800.1).

### Synthesis of CagTCaagaCCaaaaTCC (MSTN Ex3_Fox1+6) (Example 7)

The compound of Example 7 with the sequence of interest was synthesized in the same manner as described for the compound of Example 1.

The subject compound was identified by negative-ion MALDI-TOFMS (theoretical: 6439.64, found: 6439.92).

The nucleotide sequence of the subject compound is a sequence complementary to nucleotide Nos. 5142-5159 of Homo sapiens myostatin (MSTN), RefSeqGene (LRG_200) on chromosome 2 (Gene Bank accession No. NG_009800.1).

### Synthesis of CaCagTCaagaCCaaaaT (MSTN Ex3_Fox1+4) (Example 8)

The compound of Example 8 with the sequence of interest was synthesized in the same manner as described for the compound of Example 1.

The subject compound was identified by negative-ion MALDI-TOFMS (theoretical: 6437.58, found: 6437.93).

The nucleotide sequence of the subject compound is a sequence complementary to nucleotide Nos. 5144-5161 of Homo sapiens myostatin (MSTN), RefSeqGene (LRG_200) on chromosome 2 (Gene Bank accession No. NG_009800.1).

### Synthesis of aTCaCagTCaagaCCaaa (MSTN Ex3_Fox1+2) (Example 9)

The compound of Example 9 with the sequence of interest was synthesized in the same manner as described for the compound of Example 1.

The subject compound was identified by negative-ion MALDI-TOFMS (theoretical: 6437.58, found: 6437.82).

The nucleotide sequence of the subject compound is a sequence complementary to nucleotide Nos. 5146-5163 of Homo sapiens myostatin (MSTN), RefSeqGene (LRG_200) on chromosome 2 (Gene Bank accession No. NG_009800.1).

### Synthesis of gCTCaTCaCagTCaagaC (MSTN Ex3_Fox1-2) (Example 10)

The compound of Example 10 with the sequence of interest was synthesized in the same manner as described for the compound of Example 1.

The subject compound was identified by negative-ion MALDI-TOFMS (theoretical: 6458.60, found: 6458.70).

The nucleotide sequence of the subject compound is a sequence complementary to nucleotide Nos. 5150-5167 of Homo sapiens myostatin (MSTN), RefSeqGene (LRG_200) on chromosome 2 (Gene Bank accession No. NG_009800.1).

### Synthesis of gTgCTCaTCaCagTCaag (MSTN Ex3_Fox1-4) (Example 11)

The compound of Example 11 with the sequence of interest was synthesized in the same manner as described for the compound of Example 1.

The subject compound was identified by negative-ion MALDI-TOFMS (theoretical: 6475.50, found: 6475.67).

The nucleotide sequence of the subject compound is a sequence complementary to nucleotide Nos. 5152-5169 of Homo sapiens myostatin (MSTN), RefSeqGene (LRG_200) on chromosome 2 (Gene Bank accession No. NG_009800.1).

### Synthesis of gagTgCTCaTCaCagTCa (MSTN Ex3_Fox1-6) (Example 12)

The compound of Example 12 with the sequence of interest was synthesized in the same manner as described for the compound of Example 1.

The subject compound was identified by negative-ion MALDI-TOFMS (theoretical: 6475.50, found: 6475.55).

The nucleotide sequence of the subject compound is a sequence complementary to nucleotide Nos. 5154-5171 of Homo sapiens myostatin (MSTN), RefSeqGene (LRG_200) on chromosome 2 (Gene Bank accession No. NG_009800.1).

### Synthesis of TCCagagCagTaaTTggC (MSTN Ex3_1) (Example 13)

The compound of Example 13 with the sequence of interest was synthesized in the same manner as described for the compound of Example 1.

The subject compound was identified by negative-ion MALDI-TOFMS (theoretical: 6489.44, found: 6487.83).

The nucleotide sequence of the subject compound is a sequence complementary to nucleotide Nos. 5260-5277 of Homo sapiens myostatin (MSTN), RefSeqGene (LRG_200) on chromosome 2 (Gene Bank accession No. NG_009800.1).

### Synthesis of TgagCaCCCaCagCggTC (MSTN Ex3_2) (Example 14)

The compound of Example 14 with the sequence of interest was synthesized in the same manner as described for the compound of Example 1.

The subject compound was identified by negative-ion MALDI-TOFMS (theoretical: 6489.70, found: 6488.90).

The nucleotide sequence of the subject compound is a sequence complementary to nucleotide Nos. 5452-5469 of Homo sapiens myostatin (MSTN), RefSeqGene (LRG_200) on chromosome 2 (Gene Bank accession No. NG_009800.1).

### Synthesis of CTCgCCgaTgTTgTaaTa (MSTN Ex3_3) (Example 15)

The compound of Example 15 with the sequence of interest was synthesized in the same manner as described for the compound of Example 1.

The subject compound was identified by negative-ion MALDI-TOFMS (theoretical: 6479.36, found: 6477.62).

The nucleotide sequence of the subject compound is a sequence complementary to nucleotide Nos. 5734-5751 of Homo sapiens myostatin (MSTN), RefSeqGene (LRG_200) on chromosome 2 (Gene Bank accession No. NG_009800.1).

### Synthesis of CaTaTgCTgCaCCaTCCC (MSTN Ex3_4) (Example 16)

The compound of Example 16 with the sequence of interest was synthesized in the same manner as described for the compound of Example 1.

The subject compound was identified by negative-ion MALDI-TOFMS (theoretical: 6449.68, found: 6450.12).

The nucleotide sequence of the subject compound is a sequence complementary to nucleotide Nos. 5965-5982 of Homo sapiens myostatin (MSTN), RefSeqGene (LRG_200) on chromosome 2 (Gene Bank accession No. NG_009800.1).

### Synthesis of TgTaggaaaaTgCaCCTg (MSTN_Ex3_5) (Example17)

The compound of Example 17 with the sequence of interest was synthesized in the same manner as described for the compound of Example 1.

The subject compound was identified by negative-ion MALDI-TOFMS (theoretical: 6487.38, found: 6486.69).

The nucleotide sequence of the subject compound is a sequence complementary to nucleotide Nos. 6122-6139 of Homo sapiens myostatin (MSTN), RefSeqGene (LRG_200) on chromosome 2 (Gene Bank accession No. NG_009800.1).

### Synthesis of CaaaggCagTgTTgTaaT (MSTN_Ex3_6) (Example 18)

The compound of Example 18 with the sequence of interest was synthesized in the same manner as described for the compound of Example 1.

The subject compound was identified by negative-ion MALDI-TOFMS (theoretical: 6488.28, found: 6488.01).

The nucleotide sequence of the subject compound is a sequence complementary to nucleotide Nos. 6322-6339 of Homo sapiens myostatin (MSTN), RefSeqGene (LRG_200) on chromosome 2 (Gene Bank accession No. NG_009800.1).

### Synthesis of CCCCTaaggTCagTaCCa (MSTN_In2 4) (Example 19)

The compound of Example 19 with the sequence of interest was synthesized in the same manner as described for the compound of Example 1.

The subject compound was identified by negative-ion MALDI-TOFMS (theoretical: 6460.66, found: 6460.14).

The nucleotide sequence of the subject compound is a sequence complementary to nucleotide Nos. 4866-4883 of Homo sapiens myostatin (MSTN), RefSeqGene (LRG_200) on chromosome 2 (Gene Bank accession No. NG_009800.1).

### Synthesis of gTTgaagTaaTaaaCTaa (MSTN In2_3) (Example 20)

The compound of Example 20 with the sequence of interest was synthesized in the same manner as described for the compound of Example 1.

The subject compound was identified by negative-ion MALDI-TOFMS (theoretical: 6454.22, found: 6454.25).

The nucleotide sequence of the subject compound is a sequence complementary to nucleotide Nos. 4998-5015 of Homo sapiens myostatin (MSTN), RefSeqGene (LRG_200) on chromosome 2 (Gene Bank accession No. NG_009800.1).

### Synthesis of ggCCTggaaaCaCTTTTC (MSTN In22) (Example 21)

The compound of Example 21 with the sequence of interest was synthesized in the same manner as described for the compound of Example 1.

The subject compound was identified by negative-ion MALDI-TOFMS (theoretical: 6478.46, found: 6478.47).

The nucleotide sequence of the subject compound is a sequence complementary to nucleotide Nos. 5037-5054 of Homo sapiens myostatin (MSTN), RefSeqGene (LRG_200) on chromosome 2 (Gene Bank accession No. NG_009800.1).

### Synthesis of TgTTaCCTTgaCCTCTaa (MSTN_Ex3_7) (Example 22)

The compound of Example 22 with the sequence of interest was synthesized in the same manner as described for the compound of Example 1.

The subject compound was identified by negative-ion MALDI-TOFMS (theoretical: 6452.38, found: 6452.23).

The nucleotide sequence of the subject compound is a sequence complementary to nucleotide Nos. 5101-5118 of Homo sapiens myostatin (MSTN), RefSeqGene (LRG_200) on chromosome 2 (Gene Bank accession No. NG_009800.1).

### Synthesis of CTTgaCCTCTaaaaaCgg (MSTN Ex3_7+6) (Example 23)

The compound of Example 23 with the sequence of interest was synthesized in the same manner as described for the compound of Example 1.

The subject compound was identified by negative-ion MALDI-TOFMS (theoretical: 6459.50, found: 6458.24).

The nucleotide sequence of the subject compound is a sequence complementary to nucleotide Nos. 5095-5112 of Homo sapiens myostatin (MSTN), RefSeqGene (LRG_200) on chromosome 2 (Gene Bank accession No. NG_009800.1).

### Synthesis of TaCCTTgaCCTCTaaaaa (MSTN Ex3_7+3) (Example 24)

The compound of Example 24 with the sequence of interest was synthesized in the same manner as described for the compound of Example 1.

The subject compound was identified by negative-ion MALDI-TOFMS (theoretical: 6430.46, found: 6429.70).

The nucleotide sequence of the subject compound is a sequence complementary to nucleotide Nos. 5098-5115 of Homo sapiens myostatin (MSTN), RefSeqGene (LRG_200) on chromosome 2 (Gene Bank accession No. NG_009800.1).

### Synthesis of gTCTgTTaCCTTgaCCTC (MSTN Ex3_7-3) (Example 25)

The compound of Example 25 with the sequence of interest was synthesized in the same manner as described for the compound of Example 1.

The subject compound was identified by negative-ion MALDI-TOFMS (theoretical: 6470.44, found: 6469.11).

The nucleotide sequence of the subject compound is a sequence complementary to nucleotide Nos. 5104-5121 of Homo sapiens myostatin (MSTN), RefSeqGene (LRG_200) on chromosome 2 (Gene Bank accession No. NG_009800.1).

### Synthesis of TgTgTCTgTTaCCTTgaC (MSTN Ex3_7-6) (Example 26)

The compound of Example 26 with the sequence of interest was synthesized in the same manner as described for the compound of Example 1.

The subject compound was identified by negative-ion MALDI-TOFMS (theoretical: 6485.28, found: 6484.44).

The nucleotide sequence of the subject compound is a sequence complementary to nucleotide Nos. 5107-5124 of Homo sapiens myostatin (MSTN), RefSeqGene (LRG_200) on chromosome 2 (Gene Bank accession No. NG_009800.1).

### Synthesis of TggTgTgTCTgTTaCCTT (MSTN Ex3_7-9) (Example 27)

The compound of Example 27 with the sequence of interest was synthesized in the same manner as described for the compound of Example 1.

The subject compound was identified by negative-ion MALDI-TOFMS (theoretical: 6502.18, found: 6500.63).

The nucleotide sequence of the subject compound is a sequence complementary to nucleotide Nos. 5110-5127 of Homo sapiens myostatin (MSTN), RefSeqGene (LRG_200) on chromosome 2 (Gene Bank accession No. NG_009800.1).

### Synthesis of CTgTTaCCTTgaCCTCTa (MSTN Ex3_7-1) (Example 28)

The compound of Example 28 with the sequence of interest was synthesized in the same manner as described for the compound of Example 1.

The subject compound was identified by negative-ion MALDI-TOFMS (theoretical: 6454.44, found: 6454.34).

The nucleotide sequence of the subject compound is a sequence complementary to nucleotide Nos. 5102-5119 of Homo sapiens myostatin (MSTN), RefSeqGene (LRG_200) on chromosome 2 (Gene Bank accession No. NG_009800.1).

### Synthesis of TCTgTTaCCTTgaCCTCT (MSTN Ex3_7-2) (Example 29)

The compound of Example 29 with the sequence of interest was synthesized in the same manner as described for the compound of Example 1.

The subject compound was identified by negative-ion MALDI-TOFMS (theoretical: 6457.40, found: 6457.37).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TgTCTgTTaCCTTgaCCT (MSTN Ex3_7-4) (Example 30)

The compound of Example 30 with the sequence of interest was synthesized in the same manner as described for the compound of Example 1.

The subject compound was identified by negative-ion MALDI-TOFMS (theoretical: 6471.34, found: 6471.68).

The nucleotide sequence of the subject compound is a sequence complementary to nucleotide Nos. 5105-5122 of Homo sapiens myostatin (MSTN), RefSeqGene (LRG_200) on chromosome 2 (Gene Bank accession No. NG_009800.1).

### Synthesis of gTgTCTgTTaCCTTgaCC (MSTN Ex3_7-5) (Example 31)

The compound of Example 31 with the sequence of interest was synthesized in the same manner as described for the compound of Example 1.

The subject compound was identified by negative-ion MALDI-TOFMS (theoretical: 6484.38, found: 6484.65).

The nucleotide sequence of the subject compound is a sequence complementary to nucleotide Nos. 5106-5123 of Homo sapiens myostatin (MSTN), RefSeqGene (LRG_200) on chromosome 2 (Gene Bank accession No. NG_009800.1).

### Synthesis of gTgTgTCTgTTaCCTTga (MSTN Ex3_7-7) (Example 32)

The compound of Example 32 with the sequence of interest was synthesized in the same manner as described for the compound of Example 1.

The subject compound was identified by negative-ion MALDI-TOFMS (theoretical: 6515.22, found: 6515.64).

The nucleotide sequence of the subject compound is a sequence complementary to nucleotide Nos. 5108-5125 of Homo sapiens myostatin (MSTN), RefSeqGene (LRG_200) on chromosome 2 (Gene Bank accession No. NG_009800.1).

### Synthesis of ggTgTgTCTgTTaCCTTg (MSTN Ex3_7-8) (Example 33)

The compound of Example 33 with the sequence of interest was synthesized in the same manner as described for the compound of Example 1.

The subject compound was identified by negative-ion MALDI-TOFMS (theoretical: 6499.22, found: 6499.59).

The nucleotide sequence of the subject compound is a sequence complementary to nucleotide Nos. 5109-5126 of Homo sapiens myostatin (MSTN), RefSeqGene (LRG_200) on chromosome 2 (Gene Bank accession No. NG_009800.1).

### Synthesis of gCTTCaaaaTCCaCagTT (MSTN Ex3_8) (Example 34)

The compound of Example 34 with the sequence of interest was synthesized in the same manner as described for the compound of Example 1.

The subject compound was identified by Q-TOF LC/MS (theoretical: 6446.35, found: 6446.24).

The nucleotide sequence of the subject compound is a sequence complementary to nucleotide Nos. 5202-5219 of Homo sapiens myostatin (MSTN), RefSeqGene (LRG_200) on chromosome 2 (Gene Bank accession No. NG_009800.1).

### Synthesis of TCTTTTaggagCgaTaaT (MSTN Ex3_9) (Example 35)

The compound of Example 35 with the sequence of interest was synthesized in the same manner as described for the compound of Example 1.

The subject compound was identified by Q-TOF LC/MS (theoretical: 6478.31, found: 6478.52).

The nucleotide sequence of the subject compound is a sequence complementary to nucleotide Nos. 5236-5253 of Homo sapiens myostatin (MSTN), RefSeqGene (LRG_200) on chromosome 2 (Gene Bank accession No. NG_009800.1).

### Synthesis of ggaTaTTTTTgTaaaaaT (MSTN_Ex3_10) (Example 36)

The compound of Example 36 with the sequence of interest was synthesized in the same manner as described for the compound of Example 1.

The subject compound was identified by Q-TOF LC/MS (theoretical: 6461.28, found: 6461.43).

The nucleotide sequence of the subject compound is a sequence complementary to nucleotide Nos. 5292-5309 of Homo sapiens myostatin (MSTN), RefSeqGene (LRG_200) on chromosome 2 (Gene Bank accession No. NG_009800.1).

### Synthesis of agaCaTCTTTgTgggagT (MSTN_Ex3_11) (Example 37)

The compound of Example 37 with the sequence of interest was synthesized in the same manner as described for the compound of Example 1.

The subject compound was identified by Q-TOF LC/MS (theoretical: 6507.31, found: 6507.26).

The nucleotide sequence of the subject compound is a sequence complementary to nucleotide Nos. 5365-5382 of Homo sapiens myostatin (MSTN), RefSeqGene (LRG_200) on chromosome 2 (Gene Bank accession No. NG_009800.1).

### Synthesis of aTaTaTTaTTTgTTCTTT (MSTN_Ex3_12) (Example 38)

The compound of Example 38 with the sequence of interest was synthesized in the same manner as described for the compound of Example 1.

The subject compound was identified by Q-TOF LC/MS (theoretical: 6443.28, found: 6443.39).

The nucleotide sequence of the subject compound is a sequence complementary to nucleotide Nos. 5410-5427 of Homo sapiens myostatin (MSTN), RefSeqGene (LRG_200) on chromosome 2 (Gene Bank accession No. NG_009800.1).

### Synthesis of aCTCTggaaaTCaTaaaa (MSTN_Ex3_13) (Example 39)

The compound of Example 39 with the sequence of interest was synthesized in the same manner as described for the compound of Example 1.

The subject compound was identified by Q-TOF LC/MS (theoretical: 6439.33, found: 6439.67).

The nucleotide sequence of the subject compound is a sequence complementary to nucleotide Nos. 5674-5691 of Homo sapiens myostatin (MSTN), RefSeqGene (LRG_200) on chromosome 2 (Gene Bank accession No. NG_009800.1).

### Synthesis of TaaTTCaTCagaaCTCaa (MSTN Ex3_14) (Example 40)

The compound of Example 40 with the sequence of interest was synthesized in the same manner as described for the compound of Example 1.

The subject compound was identified by Q-TOF LC/MS (theoretical: 6428.34, found: 6428.49).

The nucleotide sequence of the subject compound is a sequence complementary to nucleotide Nos. 5771-5788 of Homo sapiens myostatin (MSTN), RefSeqGene (LRG_200) on chromosome 2 (Gene Bank accession No. NG_009800.1).

### Synthesis of TgTaaaTaTTaaaCaaaa (MSTN Ex3_15) (Example 41)

The compound of Example 41 with the sequence of interest was synthesized in the same manner as described for the compound of Example 1.

The subject compound was identified by Q-TOF LC/MS (theoretical: 6422.31, found: 6422.37).

The nucleotide sequence of the subject compound is a sequence complementary to nucleotide Nos. 5822-5839 of Homo sapiens myostatin (MSTN), RefSeqGene (LRG_200) on chromosome 2 (Gene Bank accession No. NG_009800.1).

### Synthesis of CagCCTaTCgTaTTagCa (MSTN_Ex3_16) (Example 42)

The compound of Example 42 with the sequence of interest was synthesized in the same manner as described for the compound of Example 1.

The subject compound was identified by Q-TOF LC/MS (theoretical: 6462.35, found: 6462.34).

The nucleotide sequence of the subject compound is a sequence complementary to nucleotide Nos. 6037-6054 of Homo sapiens myostatin (MSTN), RefSeqGene (LRG_200) on chromosome 2 (Gene Bank accession No. NG_009800.1).

### Synthesis of CTggTagCCTCagaCaTT (MSTN Ex3 17) (Example 43)

The compound of Example 43 with the sequence of interest was synthesized in the same manner as described for the compound of Example 1.

The subject compound was identified by Q-TOF LC/MS (theoretical: 6478.35, found: 6478.26).

The nucleotide sequence of the subject compound is a sequence complementary to nucleotide Nos. 6055-6072 of Homo sapiens myostatin (MSTN), RefSeqGene (LRG_200) on chromosome 2 (Gene Bank accession No. NG_009800.1).

### Synthesis of CCgTTggCaTggaTTgTT (MSTN_Ex3_18) (Example 44)

The compound of Example 44 with the sequence of interest was synthesized in the same manner as described for the compound of Example 1.

The subject compound was identified by Q-TOF LC/MS (theoretical: 6512.32, found: 6512.38).

The nucleotide sequence of the subject compound is a sequence complementary to nucleotide Nos. 6019-6036 of Homo sapiens myostatin (MSTN), RefSeqGene (LRG_200) on chromosome 2 (Gene Bank accession No. NG_009800.1).

### Synthesis of gTTTTTTaTgTgaTaaaC (MSTN_Ex3_19) (Example 45)

The compound of Example 45 with the sequence of interest was synthesized in the same manner as described for the compound of Example 1.

The subject compound was identified by Q-TOF LC/MS (theoretical: 6466.29, found: 6466.19).

The nucleotide sequence of the subject compound is a sequence complementary to nucleotide Nos. 6073-6090 of Homo sapiens myostatin (MSTN), RefSeqGene (LRG_200) on chromosome 2 (Gene Bank accession No. NG_009800.1).

### Synthesis of agagaaaCTTaCTaTTTT (MSTN Ex3_20) (Example 46)

The compound of Example 46 with the sequence of interest was synthesized in the same manner as described for the compound of Example 1.

The subject compound was identified by Q-TOF LC/MS (theoretical: 6446.31, found: 6446.34).

The nucleotide sequence of the subject compound is a sequence complementary to nucleotide Nos. 6098-6115 of Homo sapiens myostatin (MSTN), RefSeqGene (LRG_200) on chromosome 2 (Gene Bank accession No. NG_009800.1).

### Synthesis of TCTTaCaTTaaagaaaaT (MSTN_Ex3 21) (Example 47)

The compound of Example 47 with the sequence of interest was synthesized in the same manner as described for the compound of Example 1.

The subject compound was identified by Q-TOF LC/MS (theoretical: 6427.32, found: 6427.14).

The nucleotide sequence of the subject compound is a sequence complementary to nucleotide Nos. 6158-6175 of Homo sapiens myostatin (MSTN), RefSeqGene (LRG_200) on chromosome 2 (Gene Bank accession No. NG_009800.1).

### Synthesis of TgaaagCCaaCCTCTaga (MSTN_Ex3_22) (Example 48)

The compound of Example 48 with the sequence of interest was synthesized in the same manner as described for the compound of Example 1.

The subject compound was identified by Q-TOF LC/MS (theoretical: 6456.36, found: 6456.24).

The nucleotide sequence of the subject compound is a sequence complementary to nucleotide Nos. 6187-6204 of Homo sapiens myostatin (MSTN), RefSeqGene (LRG_200) on chromosome 2 (Gene Bank accession No. NG_009800.1).

### Synthesis of aTaaaaaCaaaCaCCaTT (MSTN Ex3_23) (Example 49)

The compound of Example 49 with the sequence of interest was synthesized in the same manner as described for the compound of Example 1.

The subject compound was identified by Q-TOF LC/MS (theoretical: 6406.35, found: 6406.36).

The nucleotide sequence of the subject compound is a sequence complementary to nucleotide Nos. 6252-6269 of Homo sapiens myostatin (MSTN), RefSeqGene (LRG_200) on chromosome 2 (Gene Bank accession No. NG_009800.1).

### Synthesis of TTTCTaTTaTTTTaCCaT (MSTN Ex3_24) (Example 50)

The compound of Example 50 with the sequence of interest was synthesized in the same manner as described for the compound of Example 1.

The subject compound was identified by Q-TOF LC/MS (theoretical: 6428.31, found: 6428.34).

The nucleotide sequence of the subject compound is a sequence complementary to nucleotide Nos. 6356-6373 of Homo sapiens myostatin (MSTN), RefSeqGene (LRG_200) on chromosome 2 (Gene Bank accession No. NG_009800.1).

### Synthesis of aaagTaaaTaaaaaagga (MSTN Ex3_25) (Example 51)

The compound of Example 51 with the sequence of interest was synthesized in the same manner as described for the compound of Example 1.

The subject compound was identified by Q-TOF LC/MS (theoretical: 6515.37, found: 6515.22).

The nucleotide sequence of the subject compound is a sequence complementary to nucleotide Nos. 6460-6477 of Homo sapiens myostatin (MSTN), RefSeqGene (LRG_200) on chromosome 2 (Gene Bank accession No. NG_009800.1).

### Synthesis of TgTCCTTagTgTaaaaaT (MSTN Ex3_26) (Example 52)

The compound of Example 52 with the sequence of interest was synthesized in the same manner as described for the compound of Example 1.

The subject compound was identified by Q-TOF LC/MS (theoretical: 6462.31, found: 6462.37).

The nucleotide sequence of the subject compound is a sequence complementary to nucleotide Nos. 6483-6500 of Homo sapiens myostatin (MSTN), RefSeqGene (LRG_200) on chromosome 2 (Gene Bank accession No. NG_009800.1).

### Synthesis of TTCTgTgaTgCaTgaCaT (MSTN Ex3_27) (Example 53)

The compound of Example 53 with the sequence of interest was synthesized in the same manner as described for the compound of Example 1.

The subject compound was identified by Q-TOF LC/MS (theoretical: 6480.32, found: 6480.14).

The nucleotide sequence of the subject compound is a sequence complementary to nucleotide Nos. 6530-6547 of Homo sapiens myostatin (MSTN), RefSeqGene (LRG_200) on chromosome 2 (Gene Bank accession No. NG_009800.1).

### Synthesis of TCaTgTaaaaaaaTaTaa (MSTN_Ex3_28) (Example 54)

The compound of Example 54 with the sequence of interest was synthesized in the same manner as described for the compound of Example 1.

The subject compound was identified by Q-TOF LC/MS (theoretical: 6422.31, found: 6422.49).

The nucleotide sequence of the subject compound is a sequence complementary to nucleotide Nos. 6642-6659 of Homo sapiens myostatin (MSTN), RefSeqGene (LRG_200) on chromosome 2 (Gene Bank accession No. NG_009800.1).

**(Table 1)**

| Sequences of the ASOs synthesized in Examples are shown. Capital letters represent ENA nucleic acid and small letters 2'OMe. | | | |
|---|---|---|---|
| Example | Designation | Sequence | SEQ ID NO: |
| 1 | MSTN_Ex3_BP | gaaCaaTCagTaaTaTCa | 3 |
| 2 | MSTN_Ex3_SS | aaaCggaTTCTgTTTgaa | 4 |
| 3 | MSTN_Ex3_Fox1 | TCaTCaCagTCaagaCCa | 5 |
| 4 | MSTN_Ex3_LESE12SF2 | CCT gCT gaaCCTCT gggg | 6 |
| 5 | MSTN_Ex3_LESE3 | aTTgTTgaggggaaaaCC | 7 |
| 6 | MSTN_Ex3_LESE4 | TaTagCCTgTggTaCTTa | 8 |
| 7 | MSTN_Ex3_Fox1+6 | CagTCaagaCCaaaaTCC | 9 |
| 8 | MSTN_Ex3_Fox1+4 | CaCagTCaagaCCaaaaT | 10 |
| 9 | MSTN_Ex3_Fox1+2 | aTCaCagTCaagaCCaaa | 11 |
| 10 | MSTN_Ex3_Fox1-2 | gCTCaTCaCagTCaagaC | 12 |
| 11 | MSTN_Ex3_Fox1-4 | gTgCTCaTCaCagTCaag | 13 |
| 12 | MSTN_Ex3_Fox1-6 | gagTgCTCaTCaCagTCa | 14 |
| 13 | MSTN_Ex3_1 | TCCagagCagTaaTTggC | 15 |
| 14 | MSTN_Ex3_2 | TgagCaCCCaCagCggTC | 16 |
| 15 | MSTN_Ex3_3 | CTCgCCgaTgTTgTaaTa | 17 |
| 16 | MSTN_Ex3_4 | CaTaTgCTgCaCCaTCCC | 18 |
| 17 | MSTN_Ex3_5 | TgTaggaaaaTgCaCCTg | 19 |
| 18 | MSTN_Ex3_6 | CaaaggCagTgTTgTaaT | 20 |
| 19 | MSTN_In2_4 | CCCCTaaggTCagTaCCa | 21 |
| 20 | MSTN_In2_3 | gTTgaagTaaTaaaCTaa | 22 |
| 21 | MSTN_In2_2 | ggCCTggaaaCaCTTTTC | 23 |
| 22 | MSTN_Ex3_7 | TgTTaCCTTgaCCTCTaa | 24 |
| 23 | MSTN_Ex3_7+6 | CTTgaCCTCTaaaaaCgg | 25 |
| 24 | MSTN_Ex3_7+3 | TaCCTTgaCCTCTaaaaa | 26 |
| 25 | MSTN_Ex3_7-3 | gTCTgTTaCCTTgaCCTC | 27 |
| 26 | MSTN_Ex3_7-6 | TgTgTCTgTTaCCTTgaC | 28 |
| 27 | MSTN_Ex3_7-9 | TggTgTgTCTgTTaCCTT | 29 |
| 28 | MSTN_Ex3_7-1 | CTgTTaCCTTgaCCTCTa | 30 |
| 29 | MSTN_Ex3_7-2 | TCTgTTaCCTTgaCCTCT | 31 |
| 30 | MSTN_Ex3_7-4 | TgTCTgTTaCCTTgaCCT | 32 |
| 31 | MSTN_Ex3_7-5 | gTgTCTgTTaCCTTgaCC | 33 |
| 32 | MSTN_Ex3_7-7 | gTgTgTCTgTTaCCTTga | 34 |
| 33 | MSTN_Ex3_7-8 | ggTgTgTCTgTTaCCTTg | 35 |
| 34 | MSTN_Ex3_8 | gCTTCaaaaTCCaCagTT | 36 |
| 35 | MSTN_Ex3_9 | TCTTTTaggagCgaTaaT | 37 |
| 36 | MSTN_Ex3_10 | ggaTaTTTTTgTaaaaaT | 38 |
| 37 | MSTN_Ex3_11 | agaCaTCTTTgTgggagT | 39 |
| 38 | MSTN_Ex3_12 | aTaTaTTaTTTgTTCTTT | 40 |
| 39 | MSTN_Ex3_13 | aCTCTggaaaTCaTaaaa | 41 |
| 40 | MSTN_Ex3_14 | TaaTTCaTCagaaCTCaa | 42 |
| 41 | MSTN_Ex3_15 | TgTaaaTaTTaaaCaaaa | 43 |
| 42 | MSTN_Ex3_16 | CagCCTaTCgTaTTagCa | 44 |
| 43 | MSTN_Ex3_17 | CTggTagCCTCagaCaTT | 45 |
| 44 | MSTN_Ex3_18 | CCgTTggCaTggaTTgTT | 46 |
| 45 | MSTN_Ex3_19 | gTTTTTTaTgTgaTaaaC | 47 |
| 46 | MSTN_Ex3_20 | agagaaaCTTaCTaTTTT | 48 |
| 47 | MSTN_Ex3_21 | TCTTaCaTTaaagaaaaT | 49 |
| 48 | MSTN_Ex3_22 | TgaaagCCaaCCTCTaga | 50 |
| 49 | MSTN_Ex3_23 | aTaaaaaCaaaCaCCaTT | 51 |
| 50 | MSTN_Ex3_24 | TTTCTaTTaTTTTaCCaT | 52 |
| 51 | MSTN_Ex3_25 | aaagTaaaTaaaaaagga | 53 |
| 52 | MSTN_Ex3_26 | TgTCCTTagTgTaaaaaT | 54 |
| 53 | MSTN_Ex3_27 | TTCTgTgaTgCaTgaCaT | 55 |
| 54 | MSTN_Ex3_28 | TCaTgTaaaaaaaTaTaa | 56 |

### [Examples 55-63] Synthesis of Antisense Oligonucleotides (ASOs)

Antisense oligonucleotides (ASOs) as shown in Table 2 were synthesized which are variations of MSTN_Ex3_7-2 (Example 29) with locations of ENAs changed.

### Synthesis of TcTguTaCcuTgaCcTcT (MSTN Ex3_7-2a) (Example 55)

The compound of Example 55 with the sequence of interest was synthesized in the same manner as described for the compound of Example 1.

The subject compound was identified by negative-ion MALDI-TOFMS (theoretical: 6301.1, found: 6301.8).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguTACCTTgacCTCT (MSTN-_Ex3_7-2b) (Example 56)

The compound of Example 56 with the sequence of interest was synthesized in the same manner as described for the compound of Example 1.

The subject compound was identified by negative-ion MALDI-TOFMS (theoretical: 6391.3, found: 6398.1).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguuACcTugaccTcT (MSTN_Ex3_7-2c) (Example 57)

The compound of Example 57 with the sequence of interest was synthesized in the same manner as described for the compound of Example 1.

The subject compound was identified by negative-ion MALDI-TOFMS (theoretical: 6261.1, found: 6262.4).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguuaCcTugaccTcT (MSTN_Ex3_7-2d) (Example 58)

The compound of Example 58 with the sequence of interest was synthesized in the same manner as described for the compound of Example 1.

The subject compound was identified by negative-ion MALDI-TOFMS (theoretical: 6249.1, found: 6249.7).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguuAccTugaccTcT (MSTN_Ex3_7-2e) (Example 59)

The compound of Example 59 with the sequence of interest was synthesized in the same manner as described for the compound of Example 1.

The subject compound was identified by negative-ion MALDI-TOFMS (theoretical: 6235.0, found: 6237.5).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguTaCcTugaccTcT (MSTN_Ex3_7-2f) (Example 60)

The compound of Example 60 with the sequence of interest was synthesized in the same manner as described for the compound of Example 1.

The subject compound was identified by negative-ion MALDI-TOFMS (theoretical: 6275.1, found: 6275.4).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguTaCcTugacCucT (MSTN-_Ex3_7-2g) (Example 61)

The compound of Example 61 with the sequence of interest was synthesized in the same manner as described for the compound of Example 1.

The subject compound was identified by negative-ion MALDI-TOFMS (theoretical: 6275.1, found: 6275.0).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguuAcCuTgaccTcT (MSTN_Ex3_7-2h) (Example 62)

The compound of Example 62 with the sequence of interest was synthesized in the same manner as described for the compound of Example 1.

The subject compound was identified by negative-ion MALDI-TOFMS (theoretical: 6261.1, found: 6262.4).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

### Synthesis of TcTguuAcCuTgacCucT (MSTN_Ex3_7-2i) (Example 63)

The compound of Example 63 with the sequence of interest was synthesized in the same manner as described for the compound of Example 1.

The subject compound was identified by negative-ion MALDI-TOFMS (theoretical: 6261.1, found: 6263.0).

The nucleotide sequence of the subject compound is a sequence complementary to nucleotide Nos. 5103-5120 of Homo sapiens myostatin (MSTN), RefSeqGene (LRG_200) on chromosome 2 (Gene Bank accession No. NG_009800.1).

**(Table 2)**

| Example | Designation | Sequence | SEQ ID NO: |
|---|---|---|---|
| 55 | MSTN_Ex3_7-2a | TcTguTaCcuTgaCcTcT | 61 |
| 56 | MSTN_Ex3_7-2b | TcTguTACCTTgacCTCT | 62 |
| 57 | MSTN_Ex3_7-2c | TcTguuACcTugaccTcT | 63 |
| 58 | MSTN_Ex3_7-2d | TcTguuaCcTugaccTcT | 63 |
| 59 | MSTN_Ex3_7-2e | TcTguuAccTugaccTcT | 63 |
| 60 | MSTN_Ex3_7-2f | TcTguTaCcTugaccTcT | 64 |
| 61 | MSTN_Ex3_7-2g | TcTguTaCcTugacCucT | 65 |
| 62 | MSTN_Ex3_7-2h | TcTguuAcCuTgaccTcT | 66 |
| 63 | MSTN_Ex3_7-2i | TcTguuAcCuTgacCucT | 67 |

### [Test Example]

### Experimental Methods

### Evaluation of MSTN mRNA

Changes in the splicing pattern of MSTN mRNA upon introduction of ASOs were evaluated by RT-PCR using human rhabdomyosarcoma cells (CRL-2061, ATCC).

### Cell Culture

Human rhabdomyosarcoma cells (CRL-2061, ATCC) were cultured in RPMI 1640 medium (#30263-95, Nacalai Tesque) containing 10% FBS (10270-106, Gibco). Human myoblast cells (Wada et al. Development 2002, 129; 2987-2995) were cultured in DMEM medium (08458-16, Nacalai Tesque) containing 20% FBS (10270-106, Gibco) and 2% Ultroser G (15950-017, Pall Life Sciences).

### ASO Transfection

1) Two microliters of each ASO (adjusted to a concentration of 50 pmol/µl with Nuclease-Free Water (#AM9932, Thermo Fisher Scientific)) was mixed with 100 µl of Opti-MEM medium (#31985070, Thermo Fisher Scientific). For control (without ASO treatment), 2 µl of Nuclease-Free Water was mixed with the medium.
2) In a separate tube, 4 µl of Lipofectamine^{™} 3000 Transfection Reagent (#L3000015, Thermo Fisher Scientific) was mixed with 100 µl of Opti-MEM medium.
3) Liquid from 1) and liquid from 2) were mixed and left at room temperature for 15 min.
4) Human rhabdomyosarcoma cells (CRL-2061, ATCC) cultured in 12-well plates were washed with PBS once. Then, 800 µl of Opti-MEM medium was added to each well.
5) Liquid from 3) was added to the cells of 4) above (final concentration of ASO: 100 nM), and the cells were cultured at 37°C under 5% CO₂ for 3 hrs. Then, the medium was changed to RPMI 1640 medium containing 10% FBS, and culture was further continued.

### Preparation of RNA

1) Cells transfected with each ASO were cultured for 24 hrs and washed with PBS once. Then, 300 µl of RNA extraction reagent in High Pure RNA Isolation Kit (#11828665001, Roche Life Science) was added to the cells.
2) After leaving the cells at room temperature for 10 min, the RNA extraction reagent in each well was collected into a tube.
3) RNA was extracted according to the protocol of High Pure RNA Isolation Kit. Finally, 50 µl of RNA solution was obtained.

### Reverse Transcription Reaction

1) To 500 ng of RNA, Random primers (#48190011, Thermo Fisher Scientific) and dNTP Mixture (each 2.5 mM, Takara) were added. The resultant solution was incubated at 65°C for 5 min and at 25°C for 10 min.
2) To the reaction mixture of 1), M-MLV Reverse Transcriptase (#28025013, Thermo Fisher Scientific), RNaseOUT^{™} Recombinant Ribonuclease Inhibitor (#10777-019, Thermo Fisher Scientific), DTT (attached to M-MLV) and 5x First Strand Buffer (attached to M-MLV) were added. The resultant reaction mixture was incubated at 37°C for 55 min and at 70°C for 10 min to thereby obtain cDNA.

### PCR Reaction and Confirmation of Reaction Products

1) To 2 µl of the resultant cDNA, 1 µl of primer MSTN Ex1F1 (5'-agattcactggtgtggcaag-3': SEQ ID NO: 57), 1 µl of primer MSTN_R2 (5'-tgcatgacatgtctttgtgc-3': SEQ ID NO: 58), 0.1 µl of TaKaRa Ex Taq DNA polymerase (#RR001A, Takara), 1.6 µl of dNTP Mixture (each 2.5 mM), 2 µl of 10x Ex Taq Buffer and 12.3 µl of Nuclease-Free Water were added.
2) The resultant mixture was heated at 94°C for 3 min.
3) A treatment consisting of 94°C 0.5 min, 60°C 0.5 min and 72°C 1.5 min was performed through 30 cycles.
4) Finally, the reaction mixture was heated at 72°C for 3 min.
5) The PCR products were electrophoresed on 2% agarose gel (#318-01195, Nippon Gene) and quantified with Image J (NIH, http://imagej.nih.gov/ij/). Further, the PCR products were electrophoresed and quantified with Agilent2100 bioanalyzer electrophoresis system (Agilent Technology).
6) Above operations 1) to 5) were performed on GAPDH using primer GAPDH H_F (5'-cccttcattgacctcaac-3': SEQ ID NO: 59) and primer GAPDH H_R (5'-ttcacacccatgacgaac-3': SEQ ID NO: 60). It should be noted that above operation 3) was performed through 18 cycles.

### Confirmation of Expression of Myostatin Protein

The expression of myostatin protein after ASO administration was confirmed by Western blotting. Briefly, ASO was introduced into human rhabdomyosarcoma cells (CRL-2061, ATCC) using Lipofectamine 3000 Transfection Reagent (#L3000015, Thermo Fisher Scientific). Twenty-four hours after ASO administration, cells were disrupted with Cell Lysis Buffer (#9803, Cell Signaling) (supplemented with 1 mM PMSF (#8553, Cell Signaling)), and the resultant soluble fraction was obtained as a sample. Protein contained in the sample was quantified with Qubit Protein Assay Kit (#Q33211, Thermo Fisher Scientific). Samples for SDS-PAGE were prepared by mixing the above-described sample with 4x Laemmli Sample Buffer (#1610747, Bio-Rad) and thermally treating the resultant mixture. Samples for SDS-PAGE were electrophoresed on Mini-PROTEAN TGX Precast Gels 4-20% Gel (#456-1094, BIO-RAD). As a molecular weight marker, Protein Ladder One Plus, Triple-color for SDS-PAGE (#19593-25, Nacalai Tesque) was electrophoresed. For transferring proteins onto membranes, a Transblot Turbo system (Bio-Rad) was used. Protein-transferred membranes were blocked with 2% ECL Prime Blocking Agent (#RPN418, Amersham) for 1 hr at room temperature. Then, the membrane was treated with a primary antibody at 4°C overnight; the primary antibody was either one that would recognize an N-terminal site of myostatin (Anti-GDF8/Myostatin antibody, #ab71808, Abcam) or an actin-recognizing antibody (β-Actin antibody (C4), #sc-47778, Santa Cruz Biotechnology). Subsequently, the membrane was treated with a secondary antibody at room temperature for 1 hr; the secondary antibody was either HRP-labeled anti-rabbit IgG antibody (#NA934, GE) or HRP-labeled anti-mouse IgG antibody (#NA931, GE). Detection was performed with ChemiDoc XRS+ System (Bio-Rad) using Amersham ECL Select Western Blotting Detection Reagent (#RPN2235, GE).

### Analysis of Myostatin Signaling

Inhibition of myostatin signaling by ASO administration was evaluated with a measuring system for *in vitro* myostatin transcriptional activity. In this evaluation system, a reporter gene having a luciferase gene located downstream of a Smad-binding sequence (SBE4-Luc plasmid, #16495, Addgene) is introduced into cells. Myostatin signaling was evaluated by measuring the luminescence of luciferase which was induced to be expressed from the plasmid. At this time, as a control for the luciferase reporter gene, a β-galactosidase expression vector (pSV-β-Galactosidase Control Vector, #E1081, Promega) was also introduced into cells simultaneously.

ASO was transfected into human rhabdomyosarcoma cells (CRL-2061, ATCC) using Lipofectamine 3000 Transfection Reagent (#L3000015, Thermo Fisher Scientific). Twenty-four hours after ASO transfection, cells were disrupted using the Reporter Lysis Buffer of Luciferase Assay System with Reporter Lysis Buffer (E4030, Promega), and the resultant soluble fraction was obtained as a sample. Protein contained in the sample was quantified with Qubit Protein Assay Kit (#Q33211, Thermo Fisher Scientific). Luciferase activity was evaluated by measuring luciferase luminescence signal with a multi-label plate reader ARVO X3 (PerkinElmer) using, as a substrate, the Luciferase Assay System of Luciferase Assay System with Reporter Lysis Buffer (#E4030, Promega). β-Galactosidase activity was evaluated by performing reaction at 37°C for 1 hr with the β-Gal Assay System of β-Gal Assay System with Reporter Lysis Buffer (E2000, Promega) used as a substrate, terminating the reaction by adding 1 M sodium carbonate, leaving the reaction mixture at room temperature for 15 min, and then measuring absorption at 420 nm with a multi-label plate reader ARVO X3 (PerkinElmer). Luciferase activity was corrected with β-galactosidase activity (luciferase activity / β-galactosidase activity) and shown in relative values with the result of measurement of the extract from cells without ASO treatment being taken as 1.

### Measurement of Cell Proliferation

Proliferations of human myoblasts (Wada et al. Development 2002, 129; 2987-2995) and human rhabdomyosarcoma cells (CRL-2061, ATCC) were evaluated with Cell Counting Kit-8 (CCK-8) (347-07621; Dojindo Laboratories).

### Experimental Results

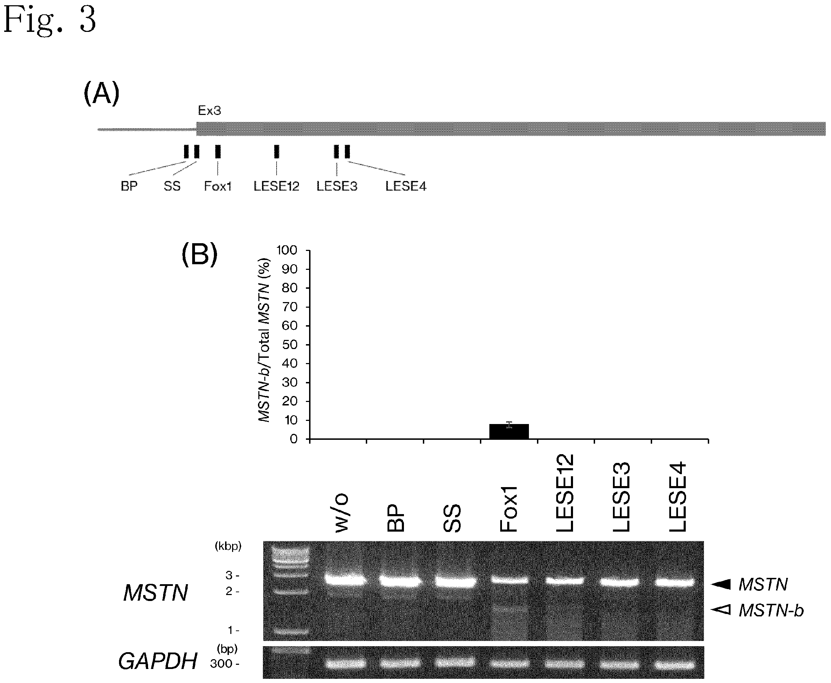

For the purpose of switching the splicing of MSTN exon 3 from production of MSTN to production of MSTN-b, the present inventors prepared 54 ASOs of 18 bases having a sequence complementary to exon 3 or intron 2 of MSTN pre-mRNA (Fig. 2). Each ASO was designed based on the prediction of binding of a splicing factor in MSTN pre-mRNA. Human rhabdomyosarcoma cells (CRL-2061) were treated with each ASO for 24 hrs and the MSTN mRNA level was subsequently examined by RT-PCR (Figs. 3-13). As a result, a significant increase in the ratio of MSTN-b to total MSTN was recognized upon treatment with MSTN_Ex3_7, MSTN_Ex3_7-1, MSTN_Ex3_7-2, MSTN_Ex3_7-3, MSTN Ex3_7-4, MSTN_Ex3_7-5, and MSTN_Ex3_7-6 (Fig. 8). These results revealed that an important sequence for splicing switch is 5'-TTAGAGGTCAAGGTAACAGACACA-3' (SEQ ID NO: 2).

As regards expression of myostatin-b, human rhabdomyosarcoma cells (CRL-2061) were treated with MSTN_Ex3_7-2 ASO for 24 hrs and examined by Western blotting. As a result, expression of myostatin-b caused by ASO treatment was confirmed.

As regards myostatin signaling, human rhabdomyosarcoma cells (CRL-2061) were treated with MSTN_Ex3_7-2 ASO for 24 hrs and examined with a measuring system for *in vitro* myostatin transcriptional activity. As a result, a decrease in specific activity of luciferase was observed upon treatment with ASO (Fig. 15). Luciferase activity in this experimental system correlates with myostatin signaling: a rise in luciferase activity means enhancement of myostatin signaling whereas a decrease in luciferase activity indicates suppression of myostatin signaling. Therefore, it has become clear that myostatin signaling is inhibited by ASO treatment.

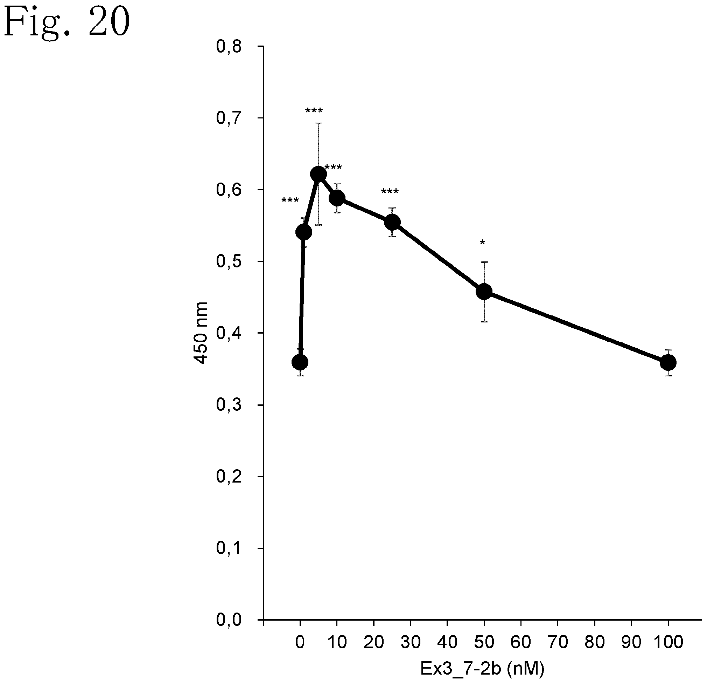

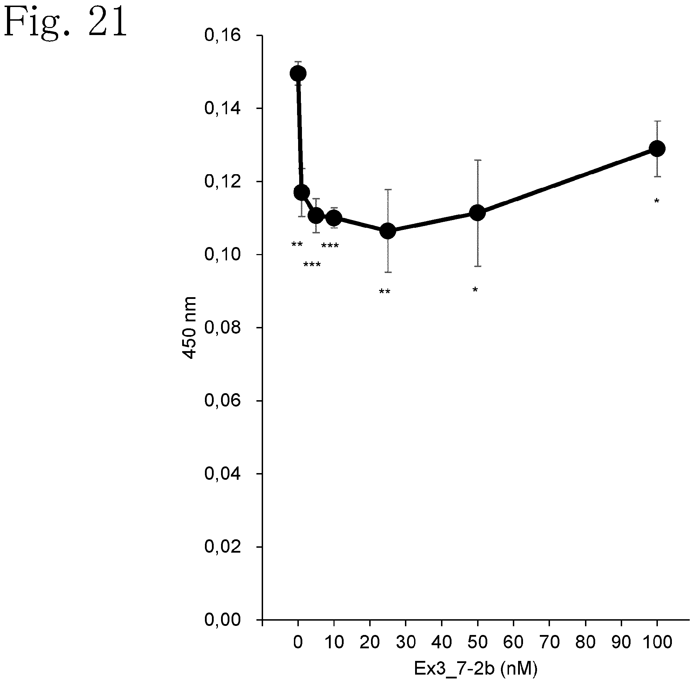

MSTN-Ex3_7-2b, a variation of MSTN-Ex3_7-2 having changed locations of ENAs showed a stronger effect on MSTN-b production than MSTN_Ex3_7-2 (Fig. 17). This effect was concentration-dependent (Fig. 18) and the ASO also caused a decrease in myostatin signaling (Fig. 19). Further, MSTN-Ex3_7-2b caused a growth promotion in human myoblast cells (Fig. 20) and a growth inhibition in human rhabdomyosarcoma cells (Fig. 21).

### Discussion

The present inventors have previously shown that expression of myostatin-b, a myostatin isoform, in cultured cells decreases myostatin signaling (WO2020/179571). This suggests that myostatin-b inhibits myostatin and promotes myogenesis.

With the ASO of the present invention, it is possible to switch the splicing of the myostatin gene from production of MSTN to production of MSTN-b. Further, the target sequence of the ASO which is critical to this splicing switch has been revealed. It is expected that factors which regulate the splicing switch would be elucidated by analyzing the splicing factors that bind to this target sequence.

When the ASO (MSTN-Ex3_7-2) of the present invention that would induceproduction of MSTN-b was administered, a decrease in MSTN and an increase in MSTN-b were confirmed at the protein level, and a decrease in myostatin signaling was also observed. It is believed that the decrease of myostatin signaling would be due to a decrease in myostatin level and the inhibition of myostatin by myostatin-b.

MSTN-Ex3_7-2b, a variation of MSTN-Ex3_7-2 having changed locations of ENAs, showed a stronger effect on MSTN-b production than MSTN_Ex3_7-2. The growth promotion in human myoblast cells as observed upon administration of MSTN-Ex3_7-2b would lead to promotion of myogenesis. Further, in view of the result of growth inhibition in human rhabdomyosarcoma cells that was caused by administration of MSTN-Ex3_7-2b, application of MSTN-Ex3_7-2b as an anticancer agent is also expected.

The greatest advantage of the ASO of the present invention is that this ASO is of a dual effect type which decreases myostatin while increasing myostatin-b by switching the splicing of the MSTN gene from production of intrinsic MSTN to production of MSTN-b (Fig. 1). In addition to that, myostatin-b as produced by ASO administration derives from human individuals, and therefore is safe with no immunogenicity. At the same time, production of myostatin-b by ASO administration utilizes the function of the gene possessed by a patient, so there is no need to introduce a large gene from outside the body.

Further, a modified nucleic acid ENA whose entry into skeletal muscle has been confirmed is used as a monomer for ASO synthesis and this removes a barrier against delivery to muscles. The present inventors have much to claim as achievements in the development of ASOs using ENA. For example, DS5141b is now under phase I/II clinical trial by Daiichi Sankyo Co., Ltd. as a therapeutic for Duchenne muscular dystrophy (DMD). This follows a preclinical study. The ASO of the present invention is prepared from the same monomer and, hence, is believed to be used in clinical scenes without any problem.

The target sequence of the ASO of the present invention is also highly conserved in cattle and pig. Therefore, the ASO is also applicable to growth promotion of these domestic animals (Fig. 16). Since inhibition of MSTN expression by ASO administration to domestic animals is not categorized as the preparation of recombinant organisms, the ASO of the present invention is easy to apply. Similarly, the ASO is also considered to be effective in dog and, hence, considered to be applicable to muscle weakness in dogs as a companion animal (Fig. 16).

All publications, patents and patent applications cited herein are incorporated herein by reference in their entirety.

### INDUSTRIAL APPLICABILITY

The present invention is applicable as a nucleic acid drug capable of allowing expression of a splicing variant of myostatin.

### SEQUENCE LISTING FREE TEXT

<SEQ ID NO: 1> shows the nucleotide sequence of exon 3 of the myostatin gene.
MSTN Ex3 1939 nt (NM_005259.3)

aatccgttt ttagaggtcaaggtaacagacaca ccaaaaagatccagaagggattttggt 60
cttgactgtgatgagcactcaacagaatcacgatgctgtcgttaccctctaactgtggat 120
tttgaagcttttggatgggattggattatcgctcctaaaagatataaggccaattactgc 180
tctggagagtgtgaatttgtatttttacaaaaatatcctcatactcatctggtacaccaa 240
gcaaaccccagaggttcagcaggcccttgctgtactcccacaaagatgtctccaattaat 300
atgctatattttaatggcaaagaacaaataatatatgggaaaattccagcgatggtagta 360
gaccgctgtgggtgctcatgagatttatattaagcgttcataacttcctaaaacatggaa 420
ggttttcccctcaacaattttgaagctgtgaaattaagtaccacaggctataggcctaga 480
gtatgctacagtcacttaagcataagctacagtatgtaaactaaaggggggaatatatgc 540
aatggttggcatttaaccatccaaacaaatcatacaagaaagttttatgatttccagagt 600
ttttgagctagaaggagatcaaattacatttatgttcctatatattacaacatcggcgag 660
gaaatgaaagcgattctccttgagttctgatgaattaaaggagtatgctttaaagtctat 720
ttctttaaagttttgtttaatatttacagaaaaatccacatacagtattggtaaaatgca 780
ggattgttatataccatcattcgaatcatccttaaacacttgaatttatattgtatggta 840
gtatacttggtaagataaaattccacaaaaatagggatggtgcagcatatgcaatttcca 900
ttcctattataattgacacagtacattaacaatccatgccaacggtgctaatacgatagg 960
ctgaatgtctgaggctaccaggtttatcacataaaaaacattcagtaaaatagtaagttt 1020
ctcttttcttcaggtgcattttcctacacctccaaatgaggaatggattttctttaatgt 1080
aagaagaatcatttttctagaggttggctttcaattctgtagcatacttggagaaactgc 1140
attatcttaaaaggcagtcaaatggtgtttgtttttatcaaaatgtcaaaataacatact 1200
tggagaagtatgtaattttgtctttggaaaattacaacactgcctttgcaacactgcagt 1260
ttttatggtaaaataatagaaatgatcgactctatcaatattgtataaaaagactgaaac 1320
aatgcatttatataatatgtatacaatattgttttgtaaataagtgtctccttttttatt 1380
tactttggtatatttttacactaaggacatttcaaattaagtactaaggcacaaagacat 1440
gtcatgcatcacagaaaagcaactacttatatttcagagcaaattagcagattaaatagt 1500
ggtcttaaaactccatatgttaatgattagatggttatattacaatcattttatattttt 1560
ttacatgattaacattcacttatggattcatgatggctgtataaagtgaatttgaaattt 1620
caatggtttactgtcattgtgtttaaatctcaacgttccattattttaatacttgcaaaa 1680
acattactaagtataccaaaataattgactctattatctgaaatgaagaataaactgatg 1740
ctatctcaacaataactgttacttttattttataatttgataatgaatatatttctgcat 1800
ttatttacttctgttttgtaaattgggattttgttaatcaaatttattgtactatgacta 1860
aatgaaattatttcttacatctaatttgtagaaacagtataagttatattaaagtgtttt 1920
cacatttttttgaaagaca 1939

ASO target sequence is shown in a box (□).

<SEQ ID NO: 2> shows the nucleotide sequences of ASO target sequence in exon 3 of the
myostatin gene.
5'-TTAGAGGTCAAGGTAACAGACACA-3'

<SEQ ID NOS: 3-56> show the sequences of ASOs synthesized in Examples 1-54.

<SEQ ID NOS: 57-60> show the sequences of primers used in Test Example.

<SEQ ID NOS: 61-67> show the sequences of ASOs synthesized in Examples 55-63.

<SEQ ID NOS: 68> shows the nucleotide sequence of MSTN-b (1860 bases in total. Start
codon (atg) and stop codon (tga) are shown in box (□)).

agattcactggtgtggcaagttgtctctcagactgtacatgcattaaaattttgcttggc 60
attactcaaaagcaaaagaaaagtaaaaggaagaaacaagaacaagaaaaaagattatat 120
tgattttaaaatcatgcaaaaactgcaactctgtgtttatatttacctgtttatgctgat 180
tgttgctggtccagtggatctaaatgagaacagtgagcaaaaagaaaatgtggaaaaaga 240
ggggctgtgtaatgcatgtacttggagacaaaacactaaatcttcaagaatagaagccat 300
taagatacaaatcctcagtaaacttcgtctggaaacagctcctaacatcagcaaagatgt 360
tataagacaacttttacccaaagctcctccactccgggaactgattgatcagtatgatgt 420
ccagagggatgacagcagcgatggctctttggaagatgacgattatcacgctacaacgga 480
aacaatcattaccatgcctacagagtctgattttctaatgcaagtggatggaaaacccaa 540
atgttgcttctttaaatttagctctaaaatacaatacaataaagtagtaaaggcccaact 600
atggatatatttgagacccgtcgagactcctacaacagtgtttgtgcaaatcctgagact 660
catcaaacctatgaaagacggtacaaggtatactggaatccgatctctgaaacttgacat 720
gaacccaggcactggtatttggcagagcattgatgtgaagacagtgttgcaaaattggct 780
caaacaacctgaatccaacttaggcattgaaataaaagctttagatgagaatggtcatga 840
tcttgctgtaaccttcccaggaccaggagaagatgggctgaatgtctgaggctaccaggt 900
ttatcacataaaaaacattcagtaaaatagtaagtttctcttttcttcaggtgcattttc 960
ctacacctccaaatgaggaatggattttctttaatgtaagaagaatcatttttctagagg 1020
ttggctttcaattctgtagcatacttggagaaactgcattatcttaaaaggcagtcaaat 1080
ggtgtttgtttttatcaaaatgtcaaaataacatacttggagaagtatgtaattttgtct 1140
ttggaaaattacaacactgcctttgcaacactgcagtttttatggtaaaataatagaaat 1200
gatcgactctatcaatattgtataaaaagactgaaacaatgcatttatataatatgtata 1260
caatattgttttgtaaataagtgtctccttttttatttactttggtatatttttacacta 1320
aggacatttcaaattaagtactaaggcacaaagacatgtcatgcatcacagaaaagcaac 1380
tacttatatttcagagcaaattagcagattaaatagtggtcttaaaactccatatgttaa 1440
tgattagatggttatattacaatcattttatatttttttacatgattaacattcacttat 1500
ggattcatgatggctgtataaagtgaatttgaaatttcaatggtttactgtcattgtgtt 1560
taaatctcaacgttccattattttaatacttgcaaaaacattactaagtataccaaaata 1620
attgactctattatctgaaatgaagaataaactgatgctatctcaacaataactgttact 1680
tttattttataatttgataatgaatatatttctgcatttatttacttctgttttgtaaat 1740
tgggattttgttaatcaaatttattgtactatgactaaatgaaattatttcttacatcta 1800
atttgtagaaacagtataagttatattaaagtgttttcacatttttttgaaagacaaaaa 1860

<SEQ ID NO: 69> shows the amino acid sequence of MSTN-b protein (251 amino acids in total).

1  60
 120
 180
 240
 251

<SEQ ID NO: 70> shows the amino acid sequence of MSTN protein (375 amino acids in total).

1 MQKLQLCVYIYLFMLIVAGPVDLNENSEQKENVEKEGLCNACTWRQNTKSSRIEAIKIQI 60
LSKLRLETAPNISKDVIRQLLPKAPPLRELIDQYDVQRDDSSDGSLEDDDYHATTETIIT 120
MPTESDFLMQVDGKPKCCFFKFSSKIQYNKVVKAQLWIYLRPVETPTTVFVQILRLIKPM 180
KDGTRYTGIRSLKLDMNPGTGIWQSIDVKTVLQNWLKQPESNLGIEIKALDENGHDLAVT 240
FPGPGEDGLNPFLEVKVTDTPKRSRRDFGLDCDEHSTESRCCRYPLTVDFEAFGWDWIIA 300
PKRYKANYCSGECEFVFLQKYPHTHLVHQANPRGSAGPCCTPTKMSPINMLYFNGKEQII 360
YGKIPAMVVDRCGCS 375

## Claims

1. An antisense oligonucleotide of 15-30 bases or a salt or a solvate thereof, wherein the antisense oligonucleotide has a nucleotide sequence complementary to a target sequence in exon 3 of the myostatin gene and is capable of allowing the expression of a splicing variant of myostatin.

2. The antisense oligonucleotide or a salt or a solvate thereof of claim 1, wherein the nucleotide sequence of exon 3 of the myostatin gene is the nucleotide sequence as shown in SEQ ID NO: 1; the target sequence in exon 3 of the myostatin gene is the sequence of the region of nucleotide Nos. 10-33 of the nucleotide sequence as shown in SEQ ID NO: 1; and the nucleotide sequence of said target sequence is represented by SEQ ID NO: 2.

3. The antisense oligonucleotide or a salt or a solvate thereof of claim 1 or 2, wherein the nucleotide sequence of the antisense oligonucleotide comprises a sequence consisting of at least 15 consecutive nucleotides in any one of the sequences as shown in SEQ ID NO: 24, 30, 31, 27, 32, 33 or 28 (wherein "t" may be "u", and "u" may be "t").

4. The antisense oligonucleotide or a salt or a solvate thereof of any one of claims 1 to 3, wherein the antisense oligonucleotide has 18 bases.

5. The antisense oligonucleotide or a salt or a solvate thereof of claim 4, wherein the nucleotide sequence of the antisense oligonucleotide is any one of the sequences as shown in SEQ ID NO: 24, 30, 31, 27, 32, 33 or 28 (wherein "t" may be "u", and "u" may be "t").

6. The antisense oligonucleotide or a salt or a solvate thereof of any one of claims 1 to 5, wherein at least one nucleotide is modified.

7. The antisense oligonucleotide or a salt or a solvate thereof of claim 6, wherein the sugar constituting the modified nucleotide is D-ribofuranose and the hydroxy group at 2'-position of D-ribofuranose is modified.

8. The antisense oligonucleotide or a salt or a solvate thereof of claim 7, wherein D-ribofuranose is 2'-O-alkylated and/or 2'-O,4'-C-alkylenated.

9. A pharmaceutical drug comprising the antisense oligonucleotide of any one of claims 1 to 8 or a pharmaceutically acceptable salt or solvate thereof.

10. The pharmaceutical drug of claim 9 for preventing and/or treating a pathological condition and/or a disease in which myostatin is involved.

11. The pharmaceutical drug of claim 10, wherein the condition and/or disease in which myostatin is involved is muscular atrophy.

12. The pharmaceutical drug of claim 11, wherein muscular atrophy is at least one selected from the group consisting of muscular dystrophy, myopathy, spinal muscular atrophy, sarcopenia and disuse muscle atrophy.

13. The pharmaceutical drug of claim 10, wherein the condition and/or disease in which myostatin is involved is a pathological condition and/or a disease in which a therapeutic effect is gained through muscle mass recovery.

14. The pharmaceutical drug of claim 13, wherein the condition and/or disease in which a therapeutic effect is gained through muscle mass recovery is at least one selected from the group consisting of cancer cachexia, diabetes, cardiovascular diseases, renal diseases and bone diseases.

15. The pharmaceutical drug of claim 14, wherein the cardiovascular disease is cardiac failure and/or arteriosclerosis; the renal disease is chronic renal failure; and the bone disease is inflammatory arthritis.

16. A food comprising the antisense oligonucleotide of any one of claims 1 to 8 or a salt or a solvate thereof that are acceptable as a food ingredient.

17. A feed comprising the antisense oligonucleotide of any one of claims 1 to 8 or a salt or solvate thereof that are acceptable as a feed ingredient.

18. An agent for promoting myocyte proliferation and/or hypertrophy, comprising the antisense oligonucleotide or a salt or a solvate thereof of any one of claims 1 to 8.

19. An agent for increasing muscle mass and/or suppressing muscle weakness, comprising the antisense oligonucleotide or a salt or a solvate thereof of any one of claims 1 to 8.

20. An agent for switching the splicing of the myostatin gene from production of myostatin to production of a splicing variant thereof, comprising the antisense oligonucleotide or a salt or a solvate thereof of any one of claims 1 to 8.

21. An agent for decreasing myostatin signaling, comprising the antisense oligonucleotide or a salt or a solvate thereof of any one of claims 1 to 8.

22. An anticancer agent comprising the antisense oligonucleotide or a salt or a solvate thereof of any one of claims 1 to 8.

23. A method of preventing and/or treating a pathological condition and/or a disease in which myostatin is involved, comprising administering to a subject an effective amount of the antisense oligonucleotide or a salt or a solvate thereof of any one of claims 1 to 8.

24. A method of promoting myocyte proliferation and/or hypertrophy, comprising administering to a subject an effective amount of the antisense oligonucleotide or a salt or a solvate thereof of any one of claims 1 to 8.

25. A method of increasing muscle mass and/or suppressing muscle weakness, comprising administering to a subject an effective amount of the antisense oligonucleotide or a salt or a solvate thereof of any one of claims 1 to 8.

26. A method of switching the splicing of the myostatin gene from production of myostatin to production of a splicing variant thereof, comprising administering to a subject an effective amount of the antisense oligonucleotide or a salt or a solvate thereof of any one of claims 1 to 8.

27. A method of decreasing myostatin signaling, comprising administering to a subject an effective amount of the antisense oligonucleotide or a salt or a solvate thereof of any one of claims 1 to 8.

28. A method of preventing and/or treating cancer, comprising administering to a subject an effective amount of the antisense oligonucleotide or a salt or a solvate thereof of any one of claims 1 to 8.

29. The antisense oligonucleotide or a salt or a solvate thereof of any one of claims 1 to 8, for use in a method of preventing and/or treating a pathological condition and/or a disease in which myostatin is involved.

30. The antisense oligonucleotide or a salt or a solvate thereof of any one of claims 1 to 8, for use in a method of promoting myocyte proliferation and/or hypertrophy.

31. The antisense oligonucleotide or a salt or a solvate thereof of any one of claims 1 to 8, for use in a method of increasing muscle mass and/or suppressing muscle weakness.

32. The antisense oligonucleotide or a salt or a solvate thereof of any one of claims 1 to 8, for use in a method of switching the splicing of the myostatin gene from production of myostatin to production of a splicing variant thereof.

33. The antisense oligonucleotide or a salt or a solvate thereof of any one of claims 1 to 8, for use in a method of decreasing myostatin signaling.

34. The antisense oligonucleotide or a salt or a solvate thereof of any one of claims 1 to 8, for use in a method of preventing and/or treating cancer.

35. Use of the antisense oligonucleotide or a salt or a solvate thereof of any one of claims 1 to 8, for preventing and/or treating a pathological condition and/or a disease in which myostatin is involved.

36. Use of the antisense oligonucleotide or a salt or a solvate thereof of any one of claims 1 to 8, for promoting myocyte proliferation and/or hypertrophy.

37. Use of the antisense oligonucleotide or a salt or a solvate thereof of any one of claims 1 to 8, for increasing muscle mass and/or suppressing muscle weakness.

38. Use of the antisense oligonucleotide or a salt or a solvate thereof of any one of claims 1 to 8, for switching the splicing of the myostatin gene from production of myostatin to production of a splicing variant thereof.

39. Use of the antisense oligonucleotide or a salt or a solvate thereof of any one of claims 1 to 8, for decreasing myostatin signaling.

40. Use of the antisense oligonucleotide or a salt or a solvate thereof of any one of claims 1 to 8, for preventing and/or treating cancer.
